# NEW EUROPEAN PATENT SPECIFICATION

(11) **EP 2 989 239 B2**
(45) Date of publication and mention of the opposition decision: **15.01.2025**
(45) Mention of the grant of the patent: 04.07.2018
(21) Application number: 14788445.6
(22) Date of filing: 23.04.2014
(51) Int. Cl.: C40B 10/00, C40B 20/04, C40B 40/02, C40B 40/08

(54) **SELECTION OF FAB FRAGMENTS USING RIBOSOMAL DISPLAY TECHNOLOGY**
AUSWAHL VON FAB-FRAGMENTEN ANHAND VON RIBOSOMALER DISPLAY-TECHNIK
SÉLECTION DE FRAGMENTS FAB AU MOYEN D'UNE TECHNOLOGIE DE PRÉSENTATION SUR RIBOSOME

(30) Priority: 25.04.2013 US 201361816075 P
(43) Date of publication of application: 02.03.2016
(73) Proprietor: Sutro Biopharma, Inc., South San Francisco, California 94080 (US)
(72) Inventor: STAFFORD, Ryan, Foster City, CA 94404 (US); THANOS, Christopher, D, Tiburon, CA 94920 (US)
(74) Representative: Murgitroyd & Company
(86) International application number: PCT/US2014/035130
(87) International publication number: WO 2014/176327

(56) References cited:
- EP-A1- 2 647 704
- US-A- 5 922 545
- US-A1- 2006 177 862
- US-A1- 2006 246 515
- US-A1- 2008 214 406
- US-A1- 2008 275 219
- US-A1- 2012 058 906
- KOJOH, K., TSUIHIHI, K., KATOH, S. AND KANAMORI, T.: "In vitro affinity maturation based on Ribosome Display System with PUREfrex", 1 January 2012 (2012-01-01), XP055070235, Retrieved from the Internet <URL:http://www.genefrontier.com/wps/wp-content/uploads/2012/05/PEGS2012_poster1.pdf> [retrieved on 20130708]
- R. L. STAFFORD ET AL: "In vitro Fab display: a cell-free system for IgG discovery", PROTEIN ENGINEERING DESIGN AND SELECTION, vol. 27, no. 4, 28 February 2014 (2014-02-28), pages 97 - 109, XP055132588, ISSN: 1741-0126, DOI: 10.1093/protein/gzu002
- YIN, G. ET AL.: "Aglycosylated antibodies and antibody fragments produced in a scalable in vitro transcription-translation system", MABS, vol. 4, 2012, pages 217 - 225
- KINDT: "Kuby Immunology SIXTH EDITION", 2007, New York, article "Chapter 4", pages: 90 - 91
- Freeman& Company N.Y
- WILSON, I. A. ET AL.: "Antibody-antigen interactions", CURR. OP. STRUCT. BIOL., vol. 3, 1993, pages 113 - 118
- LOU, J. ET AL.: "Affinity Maturation by Chain Shuffling and Site Directed Mutagenesis in Kontermann", ANTIBODY ENGINEERING, 2010, Berlin
- ANONYMOUS: "Emerging Antibody & Protein Engineering", BIOLOGICS PARTNERING FORUM, 28 April 2012 (2012-04-28), pages 1 - 8
- LEE C V, LIANG W C, DENNIS M S, EIGENBROT C, SIDHU S S, FUH G.: "High-affinity human antibodies from phage-displayed synthetic Fab libraries with a single framework scaffold.", JOURNAL OF MOLECULAR BIOLOGY, ACADEMIC PRESS, UNITED KINGDOM, vol. 340, no. 5, 23 July 2004 (2004-07-23), United Kingdom, pages 1073 - 1093, ISSN: 0022-2836, DOI: 10.1016/j.jmb.2004.05.051
- BROCKS BODO ET AL: "Generation and optimization of human antagonistic antibodies against TIMP-1 as potential therapeutic agents in fibrotic diseases", HUMAN ANTIBODIES, IOS PRESS, AMSTERDAM, NL, vol. 15, no. 4, 1 January 2006 (2006-01-01), AMSTERDAM, NL, pages 115 - 124, ISSN: 1093-2607
- FEIGE, M.J. ; HENDERSHOT, L.M. ; BUCHNER, J.: "How antibodies fold", TRENDS IN BIOCHEMICAL SCIENCES, ELSEVIER, AMSTERDAM, NL, vol. 35, no. 4, 1 April 2010 (2010-04-01), AMSTERDAM, NL, pages 189 - 198, ISSN: 0968-0004
- JIANG ET AL.: "Expression of Fab fragment of catalytic antibody 6D9 in an Escherichia coli in vitro coupled transcription/translation system", FEBS LETTERS, vol. 514, no. 2-3, 2002, pages 290 - 294, XP004347714
- WHITTAKER J.: "CELL-FREE PROTEIN SYNTHESIS: THE STATE OF THE ART", BIOTECHNOL LETT., vol. 35, no. 2, February 2013 (2013-02-01), pages 143 - 152, XP055288254

### SEQUENCE LISTING

<110> Stafford, Ryan
   Thanos, Christopher D.
<120> SELECTION OF FAB FRAGMENTS USING
   RIBOSOMAL DISPLAY TECHNOLOGY
<130> 91200-903641-005910P
<150> 61/816,075
   <151> 2013-04-25
<160> 26
<170> FastSEQ for Windows Version 4.0
<210> 1
   <211> 56
   <212> DNA
   <213> Artificial Sequence
<220>
   <223> synthetic H1a Forward primer
<400> 1
   cgcggcaagc ggttttaaca ttavtrrtwm ykmtatccac tgggtgcgtc aagcac 56
<210> 2
   <211> 56
   <212> DNA
   <213> Artificial Sequence
<220>
   <223> synthetic H1a Reverse primer
<400> 2
   gtgcttgacg cacccagtgg atakmrkway yabtaatgtt aaaaccgctt gccgcg 56
<210> 3
   <211> 56
   <212> DNA
   <213> Artificial Sequence
<220>
   <223> synthetic H1b Forward primer
<400> 3
   cgcggcaagc ggttttaaca ttavtrrtwm ykggatccac tgggtgcgtc aagcac 56
<210> 4
   <211> 56
   <212> DNA
   <213> Artificial Sequence
<220>
   <223> synthetic H1b Reverse primer
<400> 4
   gtgcttgacg cacccagtgg atccmrkway yabtaatgtt aaaaccgctt gccgcg 56
<210> 5
   <211> 77
   <212> DNA
   <213> Artificial Sequence
<220>
   <223> synthetic H2a Forward primer
<400> 5
   gggtaagggc ctggaatggg ttgstdggat tdmtccgdmt rrcggtdmta ccdactatgc 60
   ggatagcgtg aaaggcc 77
<210> 6
   <211> 77
   <212> DNA
   <213> Artificial Sequence
<220>
   <223> synthetic H2a Reverse primer
<400> 6
   ggcctttcac gctatccgca tagthggtak haccgyyakh cggakhaatc chascaaccc 60
   attccaggcc cttaccc 77
<210> 7
   <211> 77
   <212> DNA
   <213> Artificial Sequence
<220>
   <223> synthetic H2b Forward primer
<400> 7
   gggtaagggc ctggaatggg ttgstdhtat tdmtccgdmt rrcggtdmta ccdactatgc 60
   ggatagcgtg aaaggcc 77
<210> 8
   <211> 77
   <212> DNA
   <213> Artificial Sequence
<220>
   <223> synthetic H2b Reverse primer
<400> 8
   ggcctttcac gctatccgca tagthggtak haccgyyakh cggakhaata dhascaaccc 60
   attccaggcc cttaccc                                                77
<210> 9
   <211> 77
   <212> DNA
   <213> Artificial Sequence
<220>
   <223> synthetic H2c Forward primer
<400> 9
   gggtaagggc ctggaatggg ttgstgaaat tdmtccgdmt rrcggtdmta ccdactatgc 60
   ggatagcgtg aaaggcc                                                77
<210> 10
   <211> 77
   <212> DNA
   <213> Artificial Sequence
<220>
   <223> synthetic H2c Reverse primer
<400> 10
   ggcctttcac gctatccgca tagthggtak haccgyyakh cggakhaatt tcascaaccc 60
   attccaggcc cttaccc                                                77
<210> 11
   <211> 80
   <212> DNA
   <213> Artificial Sequence
<220>
   <223> synthetic H3a Forward primer
<220>
   <221> variation
   <222> 34, 35, 37, 38, 40, 41, 43, 44, 46, 47, 49, 50, 52, 53, 55, 56
   <223> n = a or g or c or t
<400> 11
   gcagaggaca ctgccgtcta ctattgtgcg cgcnnknnkn nknnknnknn knnknnkatg 60
   gactactggg gccagggtac                                             80
<210> 12
   <211> 80
   <212> DNA
   <213> Artificial Sequence
<220>
   <223> synthetic H3a Reverse primer
<220>
   <221> variation
   <222> 25, 26, 28, 29, 31, 32, 34, 35, 37, 38, 40, 41, 43, 44, 46,
   47
<223> n = a or g or c or t
<400> 12
   gtaccctggc cccagtagtc catmnnmnnm nnmnnmnnmn nmnnmnngcg cgcacaatag 60
   tagacggcag tgtcctctgc                                              80
<210> 13
   <211> 82
   <212> DNA
   <213> Artificial Sequence
<220>
   <223> synthetic H3b Forward primer
<220>
   <221> variation
   <222> 44, 45, 47, 48, 50, 51, 53, 54, 56, 57, 59, 60, 62, 63, 65,
   66
   <223> n = a or g or c or t
<400> 13
   gcagaggaca ctgccgtcta ctattgtgcg cgcnnknnkn nknnknnknn knnknnkttt 60
   gactactggg gccagggtac gc                                           82
<210> 14
   <211> 82
   <212> DNA
   <213> Artificial Sequence
<220>
   <223> synthetic H3b Reverse primer
<220>
   <221> variation
   <222> 27, 28, 30, 31, 33, 34, 36, 37, 39, 40, 42, 43, 45, 46, 48,
   49
<223> n = a or g or c or t
<400> 14
   gcgtaccctg gccccagtag tcaaamnnmn nmnnmnnmnn mnnmnnmnng cgcgcacaat 60
   agtagacggc agtgtcctct gc                                           82
<210> 15
   <211> 29
   <212> DNA
   <213> Artificial Sequence
<220>
   <223> synthetic HER2-FAB-HC-STOP reverse primer
<400> 15
   ttattaacaa caagatttcg gctccacct 29
<210> 16
   <211> 24
   <212> DNA
   <213> Artificial Sequence
<220>
   <223> synthetic HER2-FAB-LC-STOP reverse primer
<400> 16
   ttattagcac tcaccgcggt taaa 24
<210> 17
   <211> 28
   <212> DNA
   <213> Artificial Sequence
<220>
   <223> synthetic FAB forward primer
<400> 17
   atggaagttc aattagtaga aagcggcg 28
<210> 18
   <211> 25
   <212> DNA
   <213> Artificial Sequence
<220>
   <223> synthetic FAB reverse primer
<400> 18
   acaagatttc ggctccacct tcttg 25
<210> 19
   <211> 25
   <212> DNA
   <213> Artificial Sequence
<220>
   <223> synthetic Fc forward primer
<400> 19
   caagaaggtg gagccgaaat cttgt 25
<210> 20
   <211> 22
   <212> DNA
   <213> Artificial Sequence
<220>
   <223> synthetic Fc reverse primer
<400> 20
   cttacccggg gacagggaca ag 22
<210> 21
   <211> 35
   <212> DNA
   <213> Artificial Sequence
<220>
   <223> synthetic HC forward primer
<400> 21
   tatacatatg gaagttcaat tagtagaaag cggcg 35
<210> 22
   <211> 32
   <212> DNA
   <213> Artificial Sequence
<220>
   <223> synthetic HC reverse primer
<400> 22
   ttttgtcgac cttacccggg gacagggaca ag 32
<210> 23
   <211> 20
   <212> DNA
   <213> Artificial Sequence
<220>
   <223> synthetic Univ TolA reverse primer
<400> 23
   ttcagttgcc gctttctttc 20
<210> 24
   <211> 35
   <212> DNA
   <213> Artificial Sequence
<220>
   <223> synthetic HER2 HC-FAB Inner forward primer
<400> 24
   gagatatatc catggaagtt caattagtag aaagc 35
<210> 25
   <211> 29
   <212> DNA
   <213> Artificial Sequence
<220>
   <223> synthetic HER2 HC-FAB Inner reverse primer
<400> 25
   gaggcgatat aaagcttaca agatttcgg 29
<210> 26
   <211> 18
   <212> DNA
   <213> Artificial Sequence
<220>
   <223> synthetic TolA Internal everse primer
<400> 26
   ctttggcggc ttctgctt 18

## Description

### FIELD OF THE INVENTION

This invention relates to methods for selecting Fab fragments using ribosomal display technology.

### BACKGROUND OF THE INVENTION

Antibody fragments offer many advantages over full immunoglobulin molecules when used as an active agent in a therapeutic regimen. For example, being of smaller size than their full size immunoglobulin counterparts, antibody fragments can achieve higher levels of tissue penetration when administered to a patient in need thereof, and therefore higher therapeutic efficacy than a larger immunoglobulin molecule of comparable antigen specificity. Further, due to this smaller size, antibody fragments are often more easily and economically producible than their full immunoglobulin counterparts of comparable antigen specificity.

Techniques for selecting and identifying antibody fragments such as heterodimeric Fab fragments include a number of display technologies. Ribosomal display is a cell-free system using the principle of coupling phenotype (protein) to genotype (encoding nucleic acid such a DNA or RNA) for the *in vitro* selection of proteins from large libraries (Mattheakis et al., Proc. Natl. Acad. Sci. USA, 91:9022-9026 (1994); Hanes et al., Proc. Natl. Acad. Sci. USA, 94:4937-4942 (1997); He et al., Nucleic Acids Res., 25(24):5132-4 (1997)).

Prokaryotic and eukaryotic ribosomal display systems have been used for the selection of peptides, single-chain antibodies (*e.g*., scFvs), enzymes, stable protein scaffolds and other ligand-binding domains (He et al., Brief Funct. Genomic Proteomic, 1(2):204-12 (2002)). Efficient display methods for producing and selecting Fab fragment antibodies from very large libraries (*e.g*., between about 10³ to 10⁵ members, preferably 10⁵ to 10¹⁶ members) are needed.

Despite the expressed suggestions in the prior art to avoid multichain assembly using ribosomal display systems (*see, e.g.,* Simuda et al., Nucleic Acids Research, 37(22):e147 (2009); Ponsel et al., Molecules, 16:3675-3700 (2011)), it has been surprisingly found to work efficiently. Prior to our work, it was thought that the kinetics of chain association were too slow, relative to that of mRNA degradation, to permit selection of multichain proteins such as Fab fragments.

Kojoh et al., 2012, "In vitro affinity maturation based on Ribosome Display System with PUREfrex", retrieved from the Internet: URL:http://www.genefrontier.com/wps/wp-content/uploads/2012/05/PEGS2012_poster1.pdf, discloses *in vitro* affinity maturation based on a ribosome display system.

### BRIEF SUMMARY OF THE INVENTION

The invention provides a method for selecting a Fab fragment of interest from a library of Fab fragments comprising variable heavy (V_{H}) chain domains and variable light (V_{L}) chain domains. The method comprises: (i) generating a first library of DNA encoding V_{H} chain domains where the library comprises at least 10² different library members, each member having a different base sequence; (ii) generating a second library of one V_{L} chain domain member; (iii) transcribing the first library of DNA to RNA; (iv) translating the RNA of the first library in a cell free protein synthesis system to generate a ribosomal display reaction system comprising a population of complexes comprising a strand of an RNA molecule, a ribosome and V_{H} chain domains to generate a library of Fab fragment members where library members comprise a complex comprising a V_{H} chain, in association with a V_{L} chain domain where the V_{H} chain is associated with an RNA molecule and a ribosome, wherein the step of translating the RNA of the first library and generation of the V_{L} chain domains of the second library occur separately such that the generated V_{L} chain domains of the second library are added to the step of translating the RNA of the first library; and (v) selecting the Fab fragments of interest from the library of Fab fragments.

In some embodiments, the ribosome is separated from the library of Fab fragment members prior to the selecting step. In some embodiments, the selecting step comprises capturing the Fab fragments to an immobilized antigen.

In some embodiments, the step of generating the second library further comprises the step of transcribing DNA encoding the member of the second library. In some embodiments, the step of generating the variable chain of the second library comprises the step of expressing nucleic acid encoding the variable chain.

In some embodiments, the first library has between 10³ and 10⁵ members, *e.g*., 10³, 10⁴, 10⁵ variable chain members. In some embodiments, the first library has between 10⁵ and 10¹⁶ members, *e.g*., 10⁵, 10⁶, 10⁷, 10⁸, 10⁹, 10¹¹, 10¹¹, 10¹², 10¹³, 10¹⁴, 10¹⁵, or 10¹⁶ variable chain members.

In some embodiments, each member of the first library does not have a functional stop codon. In some embodiments, each member of the first library can have a functional stop codon.

The first library is a population of DNA members encoding V_{H}.

The second library is combined to the population of complexes of the first library when the complexes contains a strand of an RNA molecule, a ribosome, and a variable chain domain encoded by the RNA molecule.

In some embodiments, the RNAs of the Fab fragments selected from step (v) are amplified and the amplified products are combined with a library and a cell free, ribosomal display reaction system to generate a population of complexes comprising a strand of RNA, a ribosome and Fab fragments that are enriched for binding to the antigen used to capture the Fab fragments of step (v).

In some embodiments, the cell free protein synthesis (CFPS) system comprises an oxidative phosphorylation reaction producing ATP. In other embodiments, the CFPS system comprises a reconstituted ribosome system. In yet other embodiments, the CFPS system comprises a bacterial extract with associated co-factors.

The V_{H} chain domain is a member of a library of V_{H} chain domains such that each member has a variable chain associated with an RNA molecule encoding the variable chain, wherein the variable chain has a primary amino acid sequence different from the variable chains of the other members of the library.

### BRIEF DESCRIPTION OF THE DRAWINGS

FIG. 1A shows that to display functional heterodimeric Fab domains from ribosomes two DNA templates are used for transcription: 1) a "Display Template"lacking a stop codon at the end of a disordered TolA spacer, and 2) an " Expression Template" with a stop codon.

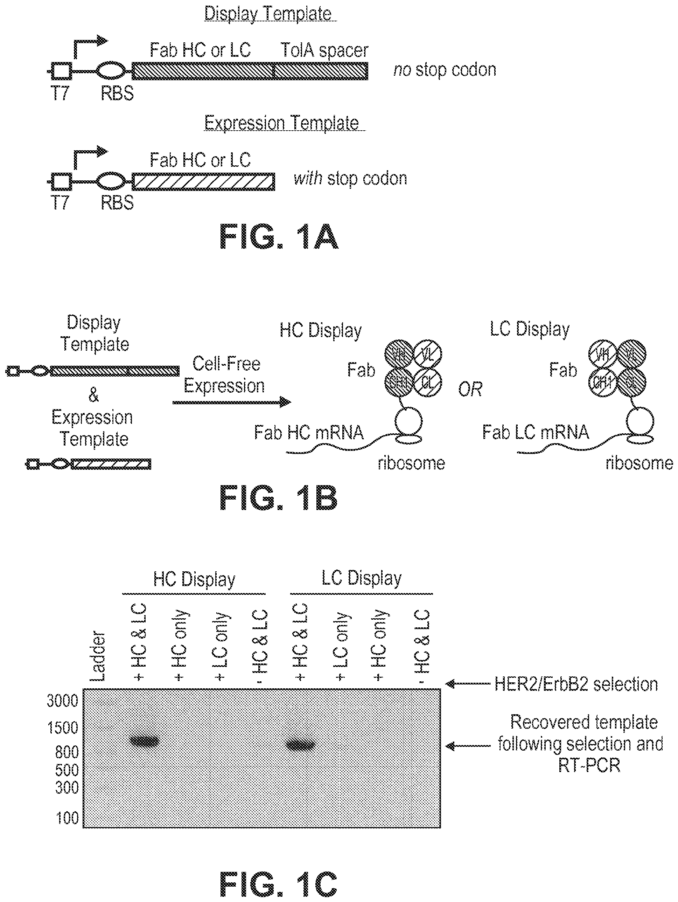

FIG. 1B shows a mixture of a display template and its complementary expression template co-expressed in a cell-free translation mixture enables the formation of a ternary complex of the functional Fab, ribosome, mRNA complex. For Fab heavy chain (HC) display, the HC display template is co-expressed with the Fab light chain (LC) expression template which enables selection of a HC library. Alternatively, co-expression of the LC display template with the HC expression template enables LC display. FIG. 1C shows co-expression of *both* the Herceptin Fab HC and LC display and expression templates in either the HC or LC display format is required for significant recovery of the template following selection with HER2/ErbB2 extra-cellular domain (ECD). This is consistent with assembly of functional heterodimeric Fabs on stalled ribosomes as the models in FIG. 1B suggest.
FIG. 2 shows the binding of selected Fab fragments to CEA after being converted to IgGs.

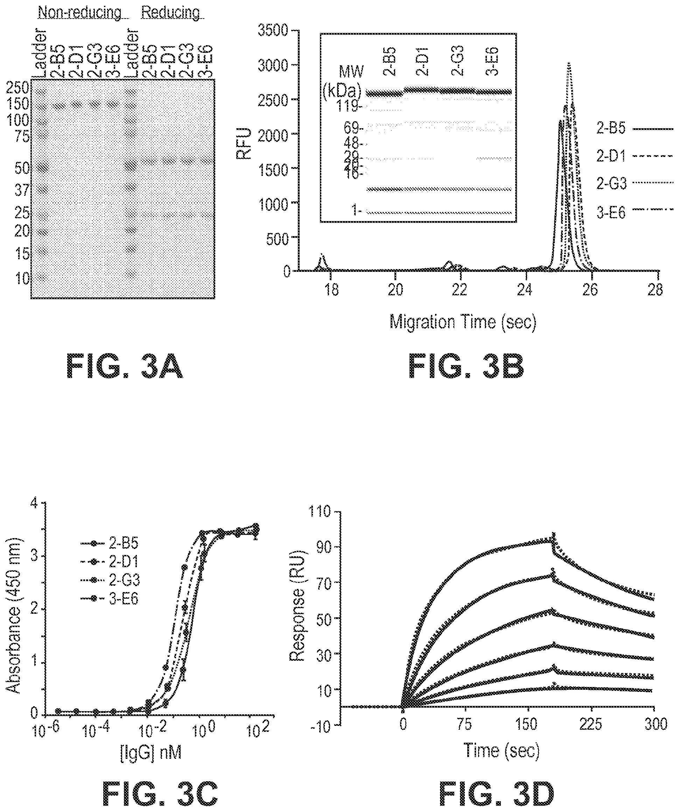

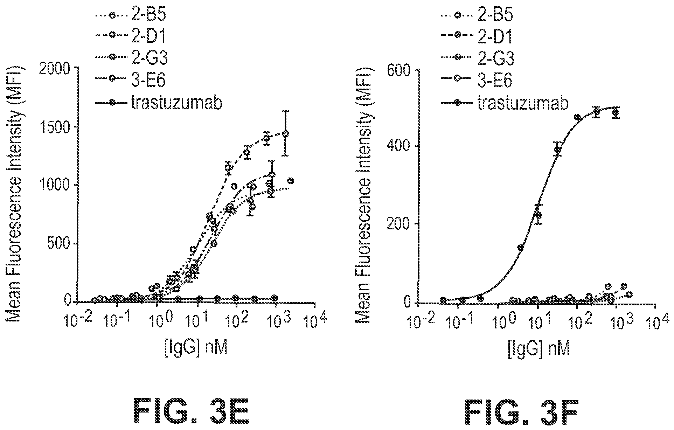

FIG. 3 shows characterization data for several IgGs reformatted from selected Fabs using the PURE system. FIG.3A shows the purified anti-CEA IgGs on a non-reducing SDS-PAGE gel. FIG. 3B illustrates the high purity of the selected IgGs as assessed by protein assay (Caliper/Perkin Elmer, Waltham, MA). The inset shows that the same sample traces where the amount of protein is indicated by the band intensity. FIG. 3C shows that the purified anti-CEA IgGs have sub-nM EC₅₀ values for CEA, as determined by ELISA. FIG. 3D depicts an example of a Biacore (GE Healthcare Biosciences, Pittsburgh, PA) surface plasmon resonance trace for the 2-G3 anti-CEA IgG (dotted lines) and global fit to a bivalent binding model (black lines). FIG. 3E shows that the selected IgGs shows apparent high affinities on MKN45 cells which express high levels of CEA, while the wild-type Herceptin (trastuzumab) IgG does not. FIG. 3F shows that the wild-type trastuzumab IgG binds to the ErbB2/HER2 expressing SKBR3 cells, but the selected CEA-specific IgGs do not.
FIG. 4 shows ELISA data of selected Fabs in extract reformatted into IgGs. Hashed bars indicated specific binding to the target antigen (*e.g*., CEA) with respect to streptavidin (grey bars).

### DETAILED DESCRIPTION OF THE INVENTION

The method provided herein is for ribosomal display and selection of Fab fragments in a cell free protein synthesis system. The method involves construction of nucleic acid libraries, formation of Fab fragment complexes from variable chain libraries (*e.g.*, V_{H} and V_{L} libraries), and selection of Fab fragment complex that specifically binds a target antigen. The nucleic acid library (first library) can be made by synthesizing a DNA library of diverse sequences (of at least 10² members, *e.g*., from about 10³ to about 10⁵, from about 10⁵ to about 10¹⁶) that are then transcribed to produce a pool of mRNAs. Ribosomal display in a cell-free protein synthesis system can be used to generate the encoded proteins, which can form Fab fragment complexes with a second library of variable chain domains. The Fab fragment complexes can be selected for binding to an immobilized target antigen. mRNA encoding the selected complexes can be used to make cDNA, which can then be amplified and the process may be repeated to enrich the population of coding sequences for Fab fragments of interest.

The open nature of completely *in vitro* selection technologies has been thought to preclude the direct selection of multimeric proteins such as Fabs (Sumida et al., Nucleic Acids Res., 37,:e147 (2009); Ponsel et al., Molecules, 16:3675-3700 (2011)), which more closely resemble IgGs than scFvs, presumably because it has been thought that each protein chain must always maintain association with its own transcript. Provided herein is a novel approach for selecting large libraries of multimeric proteins such as Fabs that should apply to all completely *in vitro* selection technologies. This is achieved by simply maintaining the genotype-phenotype linkage for only one of the chains in a multi-chain protein.

### I. Definitions

Unless defined otherwise, technical and scientific terms used herein have the same meaning as commonly understood by a person of ordinary skill in the art. See, *e.g.,* Lackie, DICTIONARY OF CELL AND MOLECULAR BIOLOGY, Elsevier (4th ed. 2007); Sambrook et al., MOLECULAR CLONING, A LABORATORY MANUAL, Cold Springs Harbor Press (Cold Springs Harbor, NY 1989); Ausubel et al., CURRENT PROTOCOLS IN MOLECULAR BIOLOGY, John Wiley and Sons (Hoboken, NY 1995). The term "a" or "an" is intended to mean "one or more." The term "comprise" and variations thereof such as "comprises" and "comprising," when preceding the recitation of a step or an element, are intended to mean that the addition of further steps or elements is optional and not excluded. Any methods, devices and materials similar or equivalent to those described herein can be used in the practice of this invention. The following definitions are provided to facilitate understanding of certain terms used frequently herein and are not meant to limit the scope of the present disclosure.

The term "peptide," "protein," and "polypeptide" are used herein interchangeably and refer to a polymer of amino acid residues. The terms apply to amino acid polymers in which one or more amino acid residue is an artificial chemical mimetic of a corresponding naturally occurring amino acid, as well as to naturally occurring amino acid polymers and non-naturally occurring amino acid polymers. As used herein, the terms encompass amino acid chains of any length, including full-length proteins and truncated proteins, wherein the amino acid residues are linked by covalent peptide bonds.

As used herein, the term "different library members" refers to members that are not identical in their primary amino acid sequence when protein nor in their base sequences when nucleic acid. In the context of this invention, "different primary amino acid sequence" refers to V_{H} or V_{L} chain domain peptides that physically differ from each other in that their primary amino acid sequences are different.

As used herein, the term "functional stop codon" refers to a nucleotide triplet within messenger RNA that signals a termination of translation. Stop codons signal the termination of this process by binding release factors, which cause the ribosomal subunits to disassociate, releasing the amino acid chain. In RNA the stop codons are UAG ("amber"); UAA ("ochre") and UGA ("opal"). The corresponding codons in DNA are in DNA: TAG ("amber") TAA ("ochre") TGA ("opal" or "umber"). When a template lacks a functional stop codon, one of these codons is not present.

As used herein, the term "Fab fragment" is an antibody fragment that contains the portion of the full-length antibody that results from digestion of a full-length immunoglobulin with papain, or a fragment having the same structure that is produced synthetically, *e.g.* recombinantly. A Fab fragment contains a light chain (containing a variable (V_{L}) and constant (C_{L}) region domain) and another chain containing a variable domain of a heavy chain (V_{H}) and one constant region domain portion of the heavy chain (C_{H1}).

As used herein, a F(ab')₂ fragment is an antibody fragment that results from digestion of an immunoglobulin with pepsin at pH 4.0-4.5, or a synthetically, *e.g.* recombinantly, produced antibody having the same structure. The F(ab')₂ fragment contains two Fab fragments but where each heavy chain portion contains an additional few amino acids, including cysteine residues that form disulfide linkages joining the two fragments.

As used herein, a "variable domain" with reference to an antibody is a specific immunoglobulin (Ig) domain of an antibody heavy or light chain that contains a sequence of amino acids that varies among different antibodies. Each light chain and each heavy chain has one variable region domain (V_{L}, and, V_{H}). The variable domains provide antigen specificity, and thus are responsible for antigen recognition. Each variable region contains CDRs that are part of the antigen binding site domain and framework regions (FRs).

As used herein, antibody fragment or antibody portion with reference to a "portion thereof" or "fragment thereof" of an antibody refers to any portion of a full-length antibody that is less than full length but contains at least a portion of the variable region of the antibody sufficient to form an antigen binding site (*e.g.,* one or more CDRs) and thus retains the a binding specificity and/or an activity of the full-length antibody; antibody fragments include antibody derivatives produced by enzymatic treatment of full-length antibodies, as well as synthetically, *e.g.* recombinantly produced derivatives. Examples of antibody fragments include, but are not limited to, Fab, Fab', F(ab')₂, single-chain Fvs (scFv), Fv, dsFv, diabody, Fd and Fd' fragments (*see, e.g.,* METHODS IN MOLECULAR BIOLOGY, Vol 207: Recombinant Antibodies for Cancer Therapy Methods and Protocols (2003); Chapter 1; pp. 3-25, Kipriyanov). The fragment can include multiple chains linked together, such as by disulfide bridges and/or by peptide linkers.

Hence, an "antibody or portion thereof that is sufficient to form an antigen binding site" includes that the antibody or portion thereof contains at least 1 or 2, typically 3, 4, 5 or all 6 CDRs of the V_{H} and V_{L} sufficient to retain at least a portion of the binding specificity of the corresponding full-length antibody containing all 6 CDRs. Generally, a sufficient antigen binding site at least requires CDR3 of the heavy chain (CDRH3). It typically further requires the CDR3 of the light chain (CDRL3). As described herein, one of skill in the art knows and can identify the CDRs based on kabat or Chothia numbering (*see, e.g.,* Kabat, E. A. et al. (1991) Sequences of Proteins of Immunological Interest, Fifth Edition, U.S. Department of Health and Human Services, NIH Publication No. 91-3242, and Chothia et al. (1987) J. Mol. Biol. 196:901-917).

The term "library of DNA encoding V_{H} or V_{L} chains" refers to a collection of different DNA species or members where each member has a nucleic acid sequence that differs from the sequence of the other members of the library. The library members can differ randomly, stochiastically, or with directed mutations of particular nucleic acids. The size of the library can vary from about 100 to 10¹² members for the first library and is 1 member in the second library.
The first library of DNA encoding V_{H} chains has more members than the second library.

The term "antibody library" refers to a plurality of nucleic acid molecules (e.g., DNA or RNA) containing an open reading frame (ORF) that encodes an antibody or fragment thereof or a plurality of peptides comprising antibodies or fragments thereof. It also includes a plurality of antibody peptides and nucleic acid/antibody fusion molecules expressed from said DNA or RNA molecules.

As used herein, "population of complexes" refers to the ternary or quaternary complexes formed from during the ribosomal display process (ternary) and after the association of the V_{H} or V_{L} chain domains (quaternary). The complexity of the population is a direct reflection of the complexity of the libraries from which the complexes arose.

The term "ribosomal display reaction system" or "RDRS" refers a cell-free system using the principle of coupling phenotype (protein) to genotype (polynucleotide such a DNA, RNA, or a combination thereof) for the *in vitro* selection of proteins from large libraries to a reaction system. It includes systems that is able to yield a ternary complex of an mRNA, ribosome and corresponding protein. Non-limiting examples of a ribosomal display reaction system include ribosome display, mRNA display, CIS display and P2A display.

Ribosome display can be used for screening antibodies and fragments thereof for target antigen binding. The steps of the reaction system can include: 1) generating a DNA library and transcribing the library into an RNA library, 2) purifying the RNA and in vitro translation in a cell-free protein synthesis system, 3) allowing the ribosome complexes of the translation reaction to bind to a target antigen, 4) selecting bound ribosome complexes; and 5) isolating RNA from the complexes and reverse transcribing the transcripts to cDNA, wherein the cDNA can be amplified, sequenced and/or further modified.

As used herein, the terms "antigen"and like terms are used herein to refer to a molecule, compound, or complex that is recognized by an antibody, conjugate thereof (*e.g*., chimeric or bispecific antibodies or scFv's), or fragment thereof (*e.g*., Fab, F(ab')2, Fv, scFv, Fd, dAb and other compositions). The term can refer to any molecule that can be specifically recognized by an antibody, conjugate thereof or fragment thereof, *e.g.,* a peptide, polynucleotide, carbohydrate, lipid, chemical moiety, or combinations thereof (*e.g*., phosphorylated or glycosylated peptides, chromatin moieties, etc.).

The terms "specific for," "specifically binds," and the like refer to the binding of a molecule (*e.g*., antibody or antibody fragment) to a target (antigen, epitope, antibody target, etc.) with at least 2-fold greater affinity than non-target compounds, *e.g.,* at least 4-fold, 5-fold, 6-fold, 7-fold, 8-fold, 9-fold, 10-fold, 20-fold, 25-fold, 50-fold, or 100-fold greater affinity. For example, a Fab fragment that specifically binds, or is specific for, is a Fab fragment that will typically bind its target antigen with at least a 2-fold greater affinity than a non-target antigen.

The term "binds" with respect to an antibody target (*e.g*., antigen, analyte, immune complex), typically indicates that an antibody binds a majority of the antibody targets in a pure population (assuming appropriate molar ratios). For example, an antibody that binds a given antibody target typically binds to at least 2/3 of the antibody targets in a solution (*e.g*., 75, 80, 85, 90, 91, 92, 93, 94, 95, 96, 97, 98, 99, or 100%). One of skill will recognize that some variability will arise depending on the method and/or threshold of determining binding.

As used herein, the term "binding affinity" refers to the strength of binding between the binding site (of a Fab fragment) and a target molecule (target antigen). The affinity of a binding site X for a target molecule Y is represented by the dissociation constant (K_{d}), which is the concentration of Y that is required to occupy half of the binding sites of X present in a solution. A lower K_{d} indicates a stronger or higher-affinity interaction between X and Y and a lower concentration of ligand is needed to occupy the sites.

As used herein, the term "reaction vessel" refers to any container in which a reaction can occur in accordance with the methods provided herein. A reaction vessel includes a microcentrifuge tube or other container of the sort, a well in a microtiter plate, a spot on a glass slide, a well in a microarray, a spot on a chip (*e.g*., a lab-on-a-chip like device), *etc.*

As used herein, the term "associated with" with reference to a variable chain domain, an RNA molecule and a ribosome refers to the formation of complex comprising of a variable chain domain, an RNA molecule and a ribosome. The components of the complex do not need to be complexed to each and every other component of the complex to form a complex.

The term "nucleic acid" refers to deoxyribonucleotides or ribonucleotides and polymers thereof in either single- or double-stranded form, and complements thereof. The term "polynucleotide" refers to a linear sequence of nucleotides. The term "nucleotide" typically refers to a single unit of a polynucleotide, *i.e*., a monomer. Nucleotides can be ribonucleotides, deoxyribonucleotides, or modified versions thereof. Examples of polynucleotides contemplated herein include single and double stranded DNA, single and double stranded RNA, and hybrid molecules having mixtures of single and double stranded DNA and RNA.

The term "amino acid" refers to naturally occurring and synthetic amino acids, as well as amino acid analogs and amino acid mimetics that function similarly to the naturally occurring amino acids. Naturally occurring amino acids are those encoded by the genetic code, as well as those amino acids that are later modified, *e.g*., hydroxyproline, γ-carboxyglutamate, and O-phosphoserine. Amino acid analogs refers to compounds that have the same basic chemical structure as a naturally occurring amino acid, *e.g.,* an α carbon that is bound to a hydrogen, a carboxyl group, an amino group, and an R group, *e.g.,* homoserine, norleucine, methionine sulfoxide, methionine methyl sulfonium. Such analogs may have modified R groups (*e.g*., norleucine) or modified peptide backbones, but retain the same basic chemical structure as a naturally occurring amino acid. Amino acid mimetics refers to chemical compounds that have a structure that is different from the general chemical structure of an amino acid, but that functions similarly to a naturally occurring amino acid.

The term "cell free protein synthesis" or "CFPS" refers to the *in vitro* synthesis of nucleic acids and/or polypeptides in a reaction mix comprising biological extracts and/or defined reagents. The reaction mix will comprise a template for production of the macromolecule, e.g. DNA, mRNA, *etc.*; monomers for the macromolecule to be synthesized, *e.g.* amino acids, nucleotides, *etc*.; and co-factors, enzymes and other reagents that are necessary for the synthesis, *e.g.* ribosomes, uncharged tRNAs, tRNAs charged with natural and/or unnatural amino acids, polymerases, transcriptional factors, tRNA synthetases, *etc.*

### II. Fab fragments

A Fab fragment refers to Fab (fragment, antigen binding) region and represents that portion of an intact antibody that results from a papain digestion to produce two Fab fragments and an Fc fragment. The Fab fragment contains the antigen binding portion of the antibody called the paratope at the amino terminal end of the antibody monomer. It is made up of a variable heavy (V_{H}) chain and a dimer association with a variable light (V_{L}) chain. It is composed of one or a portion of the constant domain and one variable domain from each heavy and light chain of an antibody. The variable domain is also referred to as the FV region and is the most important region for binding to antigens. More specifically, variable loops of β-strands, three each on the light (V_{L}) and heavy (V_{H}) chains are responsible for binding to the antigen. These loops are referred to as the complementarity determining regions (CDRs). The structures of these CDRs have been clustered and classified by Chothia et al., J. Mol. Biol. 273 (4): 927-948 (1997), and more recently by North et al., J. Mol. Biol., 406(2): 228-256 (2010). In between the CDRs are the framework regions (FRs) which are relatively non-variable regions. The CDRs from the two chains of each pair are aligned by the framework regions, enabling binding to a specific epitope. The sequences of the framework regions of different light or heavy chains are relatively conserved within a species. The framework region of an antibody, that is the combined framework regions of the constituent light and heavy chains, serves to position and align the CDRs in three dimensional space.

### III. V_{H} and V_{L} chain domains

The heavy and light chain variable regions are similar in length (about 110 amino acids), although the heavy chain variable region is typically longer, *e.g*., about 120 amino acids in length. A variable region can be readily identified by sequence comparison to databases of variable region sequences, *e.g*., the V-base database (provided by the MRC Centre for Protein Engineering, Cambridge, United Kingdom (*see, e.g.,* Chothia et al., J. Mol. Biol., 227:776-798 (1992); Tomlinson et al., EMBO J, 14:4628-4638 (1995); and Williams et al., J. Mol. Biol., 264:220-232 (1996)) or the IMGT database (Lefranc,M.-P. IMGT, the international ImMunoGeneTics database. Nucleic Acids Res., 29(1):207-9 (2001)). Alternatively, a variable region can be identified using probes or primers specific for particular variable region gene family members, *e.g*., primers or probes based on antibody leader sequences or based on known variable region framework amino acid sequences. Nucleic acid sequences for designing such primers and probes are available from V-base and other databases such as the Kabat database (Kabat et al., 1991, Sequences of Proteins of Immunological Interest, Fifth Edition, NIH Publication No. 91-3242, Bethesda, MD) or are available in the art, (*e.g.,* Orlandi et al., Proc. Natl. Acad. Sci. USA, 86:3833 (1989); Sblattero et al., Immunotechnology, 3:271 (1998); or Krebber et al., J. Immunol. Methods, 201:35 (1997)).

The region of the V-region (heavy or light chain) that is encoded by a V gene is also called a V-segment. The genetic segments of FR1, CDR1, FR2, CDR2, and FR3, including CDR3 and FR4, which segments are added to the V-segment as a consequence of rearrangement of the heavy chain and light chain V-region genes endogenously during B-cell differentiation. V, D and J segments are found in Ig heavy chains, but only V and J segments are found in Ig light chains.

### IV. Construction of nucleic acid libraries (first libraries) encoding variable chain domains

The nucleic acid (DNA) library of V_{H} chain domains provided herein comprises a plurality of different V_{H} chain domains, wherein each V_{H} chain domain member comprises a nucleic acid sequence encoding a variable chain domain different from another member of the library. Each V_{H} chain domain member of the DNA library does not encode a functional stop codon.

The DNA library of V_{H} chain domains can comprise of at least 10² different library members, *e.g*., 10², 10³, 10⁴, 10⁵, 10⁶, 10⁷, 10⁸, 10⁹, 10¹⁰, 10¹¹, 10¹², 10¹³, 10¹⁴, 10¹⁵, 10¹⁶ or more different library members. Each member in the library can encode for a different amino acid sequence, and thus a different variable chain domain.

The nucleic acid sequences used to generate the nucleic acid (DNA) library of variable chain domains can be obtained from any source. For instance, the variable chain domains can be from an antibody library, such as a naive library, immune library, semi-synthetic library, or a synthetic library. A naive library can be derived from primary B-cells of non-immunized donors and can be used to produce antibodies against all types of antigens. An immune library can be derived from immunized donors and be enriched for antibodies that recognize the specific antigen used for immunization. A synthetic library can contain antibodies designed *in silico* and then synthesized by recombinant or chemical methods known in the art. A semi-synthetic library can be a combination of a naturally-derived library such as a naive or immune library and a synthetic library.

In one aspect, the DNA library can be from the antibody repertoire of any animal including, but not limited to, rodents, primates, camelids, sharks, or any transgenic animal containing a repertoire of human immunoglobulin genes. Techniques for the isolation and cloning of nucleic acids encoding the variable regions of the antibody complement of an organism are well known in the art. cDNA libraries containing nucleic acids encoding the variable regions of antibodies are commercially available, for example, libraries of human antibody variable regions generated from various immune cells, for example, peripheral blood mononuclear cells (PBMC), spleen or lymph node. In other aspects, the library can be obtained by synthesis of nucleic acids encoding variable chain domains.

In some embodiments, the method of generating the nucleic acid (DNA) library of variable chain domains comprises molecular amplification (*e.g*., PCR amplification, ligase chain reaction, strand displacement amplification, self-sustained sequence replication, nucleic acid based sequence amplification, site-directed mutagenesis, restriction enzyme digestion, ligation, cloning and any combination thereof) of the variable chain domain of interest.

In some embodiments, the DNA library of V_{H} chain domains is produced by combining a V_{H}, a D_{H} and a J_{H} human germline segment or portion thereof, or degenerate codons thereof in frame to generate a sequence of a nucleic acid molecule encoding a V_{H} chain domain or a portion thereof. A DNA library of V_{L} chain domains may be produced by combining a V_{κ} and a J_{κ} human germline segment or portion thereof, or a V_{λ} and a J_{λ} germline segment or portion thereof in frame to generate a sequence of a nucleic acid molecule encoding a V_{L} chain domain or a portion thereof or degenerate codons thereof.

To introduce additional diversity, the DNA library of the method provided herein may require the introduction of nucleic acid substitution(s) and/or deletion(s) that can result in one or more amino acid substitutions and/or deletions to the expressed variable chain domains. The DNA library of variable chain domains can be modified or mutations by standard techniques known in the art, such as PCR mutagenesis, cassette mutagenesis, site-directed mutagenesis, random point mutagenesis, mutagenesis using uracil containing templates, oligonucleotide-directed mutagenesis, phosphorothioate-modified DNA mutagenesis, mutagenesis using gapped duplex DNA, point mismatch repair, mutagenesis using repair-deficient host strains, restriction-selection and restriction-purification, deletion mutagenesis, mutagenesis by total gene synthesis, and double-strand break repair.

Modifications of the variable chain domains of the library can be generated randomly or empirically. For example, random mutation of one or more regions can increase diversity of the library, particularly where modifications are made to any of the CDR-loop regions, which contribute to the specificity and affinity of the antibody. In another example, modifications can be empirically generated using rational or semi-rational approaches. Among such empirical modifications of nucleic acid molecules encoding V_{H} and V_{L} chains contemplated herein include, but are not limited to, modifications of the CDR regions, for example for the generation of directed libraries, modifications to optimize codon usage, and/or modifications to introduce restriction sites or detectable moieties. Modifications also can include a combination of random and empirical modifications.

The nucleic acid library can contain additional sequences to facilitate the expression and screening of the encoded Fab fragments *in vitro.* In one aspect, the sequence can be a promoter to be used in conjunction with its complementary RNA polymerase for mRNA synthesis. Non-limiting examples of a promoter capable of directing synthesis from a linear, double-stranded DNA include T7, SP6 and T3 phage promoters. In another aspect, the sequence can be a 5' prime, untranslated region (5' UTR) that corresponds to the RNA sequence upstream of the translation start site. Generally, the translation start site is an AUG codon. In some instances, codons other than AUG are used in naturally-occurring coding sequences, and these codon sequences can be included in the nucleic acid sequences of members of the variable chain libraries. In other instances, the additional sequence can be a Kozak sequence. In yet other instances, the sequence can also be a polyadenylation (poly A) sequence that does not contain a stop codon. The poly A sequence can be positioned after the variable chain domain coding sequence. Such sequences are well known in the art and any such sequence is contemplated. In some embodiment, sequences (*e.g.,* of oligonucleotide amplification primers, of restriction enzyme recognition sites, *etc*.) useful to facilitate amplification of the variable chain domains are included.

In some instances, the recombinant nucleic acid sequence of the variable chain domain is subsequent cloning into a vector backbone. In some instances, the vector backbone is expressed into a host cell including a prokaryotic cell or a eukaryotic cell. Host cells include bacterial cells (*e.g., E. coli*), yeast cells, fungal cells, Archaea, algal cells, plant cells, insect cells, animal cells (*e.g*., human cells or cells in animals).

Recombinant molecules can be introduced into host cells via, for example, transformation, transfection, infection, and electroporation. Generally, standard transfection methods are used to produce bacterial, yeast, algal, insect, or animal cell lines that express large quantities of peptides.

Transformation of host cells with recombinant nucleic acid molecules that incorporate the isolated recombined variable region gene, cDNA, or synthesized DNA sequence enables generation of multiple copies of the gene. Thus, the gene can be obtained in large quantities by growing transformants, isolating the recombinant DNA molecules from the transformants and, when necessary, retrieving the inserted gene from the isolated recombinant DNA. Generally, after the expression vector is introduced into the cells, the transfected cells are cultured under conditions favoring expression of the variable domain chain, which is recovered from the culture using standard purification techniques as described herein.

In some embodiments, DNA of the variable chain domain is transcribed to RNA in a host cell and the RNA is purified by methods known in the art.

In other embodiments, RNA is prepared synthetically *in vitro* from cloned DNA using RNA polymerases (*e.g.*, RNA polymerase II, SP6 RNA polymerase, T3 RNA polymerase, T7 RNA polymerase, RNA polymerase III and/or phage derived RNA polymerases) by methods known in the art.

In yet other embodiments, RNA is produced *in vitro* from a template DNA; both transcription and translation occur in this type of CFPS reaction. In other embodiments, transcription and translation systems are coupled or complementary transcription and translation systems, which carry out the synthesis of both RNA and protein in the same reaction such as cell-free protein synthesis.

### V. Construction of other libraries (second libraries) of variable chain domains

The V_{L} chain domain library comprises 1 V_{L} chain domain member.

In the invention, the second library of the invention comprises one V_{L} chain domain member as the DNA library (first library) of the present invention encodes V_{H} chain domains.

The second library is generated by transcribing DNA encoding members of the library such as V_{L} domains. Methods for generating DNA encoding a variable chain domain and for transcribing the specific DNA are described in the previous section.

The second library comprises a peptide library, wherein the peptide library is a V_{L} chain domain library. The variable domain libraries can be obtained from antibody libraries such as naive, immune, semi-synthetic, and synthetic libraries, as described herein.

A library of variable chain domains can be created by methods known in the art, *e.g.,* de novo synthesis chemically, synthesis utilizing recombinant molecular and cellular methodologies, (*e.g., in vitro* transcription or *in vitro* transcription-translation), harvesting natural diversity from lymphocytes using PCR and *in vitro* transcription-translation, and protein purification.

The V_{H} chain domain or the V_{L} chain domain peptides can be purified from protein extracts to substantial purity using standard protein purification techniques known in the art including but not limited to, SDS-PAGE, size fraction and size exclusion chromatography, ammonium sulfate precipitation, chelate chromatography, ionic exchange chromatography or column chromatography. The V_{H} chain domain or the V_{L} chain domain can be purified to 60%, 70%, 80% purity and typically at least 90%, 91%, 92%, 93%, 94%, 95%, 96%, 97%, 98% or 99% purity. Purity can be assessed by standard methods such as by SDS-PAGE and coomassie staining.

### VI. Ribosomal display

Ribosomal display can be used to perform *in vitro* protein evolution to create proteins *(e.g.,* Fab fragments) that bind to a specific target molecule. Ribosomal display is a well-known technology useful for generating libraries. This entirely *in vitro* method allows for libraries with a diversity of 10¹⁶ members. The process results in translated proteins that are associated with their RNA progenitor which can be used, as a complex, to bind to an immobilized target molecule in a selection step. The RNA-protein complex that shows high binding affinity can then be reverse transcribed to cDNA and their sequence amplified via PCR. The process also provides for repeated cycles of iteration or protein expression. In addition, nucleic acid mutations can be introduced efficiently into the selected nucleic acid library in subsequent cycles, leading to continuous DNA diversification and thus, protein evolution. The end result is a nucleic acid sequence that can be used to create tightly binding proteins (*e.g*., Fab fragments that specifically bind to the target antigen.

In the method provided herein, a peptide of a V_{H} chain domain is displayed on the surface of a ribosome from which it has been translated. Briefly, a library of RNA molecules is translated in an *in vitro* translation system to the 3' prime end of the RNA molecule, such that the ribosome does not fall off. This is accomplished by not incorporating a functional stop codon in the RNA template. Normally, the stop codon is being recognized by release factors that trigger detachment of the nascent polypeptide from the ribosome. The peptide emerges from the ribosome, but does not fall free of the complex, in such a way that it can associate with a different variable chain (V_{L} chain domain) to form a Fab fragment. In some instances, there is an additional folding step in an oxiding environment (important for forming disulfide bonds).

In some embodiments, ribosomal display reaction is performed at 30 °C for about 3 to about 30 minutes, *e.g*., 3, 4, 5, 6, 7, 8, 9, 10, 11, 12, 13, 14, 15, 16, 17, 18, 19, 20, 21, 22, 23, 24, 25, 26, 27, 28, 29, or 30 minutes.

In some instances, the reaction includes a factor, molecule, protein, chemical or the like that stabilizes the RNA or protein encoded by the RNA of the ribosomal display reaction system. The inventors have discovered that the addition of λGam protein to the coupled transcription-translation reaction increases the stability of the RNA transcript in the complexes formed in the RDRS. In some embodiments, λGam protein is included in the ribosomal display reaction system at a final concentration of about 25 to about 80 µg/ml, *e.g*., 25, 26, 27, 28, 29, 30, 31, 32, 33, 34, 35, 36, 37, 38, 39, 40, 41, 42, 43, 44, 45, 46, 47, 48, 49, 50, 51, 52, 53, 54, 55, 56, 57, 58, 59, 60 ,61, 62, 63, 64, 65, 66, 67, 68, 69, 70 ,71, 72, 73, 74, 75, 76, 77, 78, 79, or 80µg/ml. In various embodiments, the λ GamS protein is in the cell-free translation system at a final concentration of about 25 to about 80 µg/ml, *e.g.,* 5, 26, 27, 28, 29, 30, 31, 32, 33, 34, 35, 36, 37, 38, 39, 40, 41, 42, 43, 44, 45, 46, 47, 48, 49, 50, 51, 52, 53, 54, 55, 56, 57, 58, 59, 60 ,61, 62, 63, 64, 65, 66, 67, 68, 69, 70 ,71, 72, 73, 74, 75, 76, 77, 78, 79, or 80 µg/ml. In other embodiments, λGam protein is in the ribosomal display reaction system at a final concentration of about 25 to about 60 µg/ml, *e.g*., 25, 26, 27, 28, 29, 30, 31, 32, 33, 34, 35, 36, 37, 38, 39, 40, 41, 42, 43, 44, 45, 46, 47, 48, 49, 50, 51, 52, 53, 54, 55, 56, 57, 58, 59 or 60 µg/ml. In some embodiments, the λGamS protein is in the cell-free translation system at a final concentration of about 25 to about 60 µg/ml, *e.g.,* 25, 26, 27, 28, 29, 30, 31, 32, 33, 34, 35, 36, 37, 38, 39, 40, 41, 42, 43, 44, 45, 46, 47, 48, 49, 50, 51, 52, 53, 54, 55, 56, 57, 58, 59 or 60 µg/ml.

The coupled transcription/translation reactions can be quenched (stopped) by adding, *e.g.,* magnesium acetate (*e.g.,* final concentration of 50 mM) or a washing buffer with Tween (*e.g.,* 50 mM Tris-acetate (pH 7.5), 150 mM NaCl, 50 mM MgAc, 0.05% Tween-20) supplemented with BSA and heparin. In some embodiments, the reaction is placed on ice.

The whole complex of folded Fab fragment, ribosome and RNA, which can be stable for at least 10 days at 4 °C (Schaffitzel et al., J. Immunological Methods, 231:119-135 (1999)), can then be screened against a target antigen that is recognized by the translated Fab fragment. The RNA of the selected Fab fragment determined to specifically bind to the target antigen can be reverse transcribed into single stranded cDNA which can be converted to double-stranded DNA and amplified by PCR, providing the coding sequence of the selected protein (*e.g*., variable chain domain). The reverse transcription reaction can be performed on mRNA associated in the ribosomal display complex, or the mRNA can be isolated from the ribosomal display complex then used in the reverse transcription step. Suitable methods for disruption/dissociation of ribosome complexes are known in the art and include EDTA treatment and/or phenol extraction.

In general, nucleic acid (DNA) constructs for ribosomal display contain a promoter (T7, SP6 or T3), a translation initiation signal such as a Shine-Dalgarno (prokaryotic) or Kozak (eukaryotic) sequence, initiation codon, and coding sequence of the protein (*e.g*., V_{H} chain domain). One or more nucleic acid sequences encoding one or more detection tags may be included to provide for production of a protein further comprising one or more detection tags (*e.g*., histidine tag). To enable the complete nascent protein to be displayed and fold into its active conformation, a spacer domain of at least 23-30 amino acids length can be added at the C-terminus, to allow the protein to exit completely from the ribosome. The spacer can provide a known sequence for the design of primers for RT-PCR recovery of the DNA sequences.

To remove the stop codon from DNA, a 3' primer lacking the stop codon can be used during PCR construction. Constructs designed for bacterial-based display systems can incorporate sequences containing stem-loop structures at the 5' and 3' ends of the DNA to stabilize mRNA against degradation by RNase activities in bacterial cell-free systems.

The mRNA translation system used in a method of the invention may be any suitable available system. A prokaryotic or eukaryotic translation system may be used, for example crude *E. coli* lysate (commercially available from *e.g*. Promega Corp., Madison, WI; Agilent Technologies, Santa Clara, CA; GE Healthcare Biosciences, Pittsburgh, PA; Life Technologies, Carlsbad, CA), a reconstituted ribosome system such as PURE (*see, e.g.,* Shimizu et al., Nat. Biotechnol., 19:751-755 (2001)), or cell free protein synthesis as described below.

The PURE system can include about 32 individually purified components used for *in vitro* protein biosynthesis (*e.g.,* initiation, elongation and termination). In some embodiments, the components include initiation factors (*e.g.,* IF1, IF2, IF3), elongation factors (*e.g.,* EF-G, EF-Tu, EF-Ts), release factors (*e.g.,* RF1, RF3), a termination factor (*e.g.,* RRF), 20 aminoacyl-tRNA synthetases, methionyl-tRNA transformylase, T7 RNA polymerase, ribosomes, 46 tRNAs, NTPs, creatine phosphate, 10-formyl-5,6,7,8-tetrahydrofolic acid, 20 amino acids, creatine kinase, myokinase, nucleoside-diphophate kinase and pryophosphatase.

The methods and systems described herein can use a cell lysate for *in vitro* translation of a target protein of interest. For convenience, the organism used as a source for the lysate may be referred to as the source organism or host cell. Host cells may be bacteria, yeast, mammalian or plant cells, or any other type of cell capable of protein synthesis. A lysate comprises components that are capable of translating messenger ribonucleic acid (mRNA) encoding a desired protein, and optionally comprises components that are capable of transcribing DNA encoding a desired protein. Such components include, for example, DNA-directed RNA polymerase (RNA polymerase), any transcription activators that are required for initiation of transcription of DNA encoding the desired protein, transfer ribonucleic acids (tRNAs), aminoacyl-tRNA synthetases, 70S ribosomes, N¹⁰-formyltetrahydrofolate, formylmethionine-tRNAf^{Met} synthetase, peptidyl transferase, initiation factors such as IF-1, IF-2, and IF-3, elongation factors such as EF-Tu, EF-Ts, and EF-G, release factors such as RF-1, RF-2, and RF-3, and the like.

Ribosomal display has been used to successfully generate single chain antibody fragments with high affinity for their target. Detailed description of ribosomal display is found in, *e.g.,* Hanes, J. Proc. Natl. Acad. Sci. USA, 95: 14130-14135 (1998); Schaffitzel et al., J. Immunological Methods, 231:119-135 (1999); He et al., J. Immunological Methods, 231, 105-117 (1999); Roberts RW, Current Opinion in Chemical Biology, 3: 268-273 (1999).

Without being bound to any theory, it has been discovered that the presence of the V_{L} chain assists proper folding of the V_{H} chain.

Steps of translating RNA of the first library and generating the variable chains of the second library are performed in separate reaction vessels. For instance, V_{L} chain domains of the second library can be produced in a first reaction vessel, and then added to a second reaction vessel in which ribosomal display is performed using the RNA of first library of V_{H} chains.

### VI. Other Display Methods for In Vitro Selection

Other *in vitro* library display methods can be used in the invention, such as, but not limited to, puromycin-based display (*e.g*., mRNa display or cDNA display), P2A display, and CIS display (cis-activity display).

In some embodiments, the step of selecting Fab fragments of interest from the library of Fab fragments occurs in the presence of ribosomes. In other embodiments, the step of selecting Fab fragments of interest occurs in the absence of ribosomes.

### A. mRNA display

In mRNA display, each member of the RNA library is directly attached to the protein of interest (V_{H} chains) it encodes by a stable covalent linkage to puromycin, an antibiotic that can mimic the aminoacyl end of tRNA (*see, e.g*., Robert RW and Szostak JW, Proc. Natl. Acad. Sci. USA, 94:12297-12302 (1997); Nemoto et al., FEBS Letters, 414:405-408 (1997), Tabuchi et al., FEBS Letters, 508:309-312 (2001)).

Members of the RNA library can be ligated to puromycin or a derivative thereof via a linker such as, but not limited to, a polynucleotide or a chemical linker, *e.g*., polyethylene glycol (Fukuda et al., Nucleic Acid Research, 34(19):e127 (2006)). Non-limiting examples of puromycin derivatives include puromycin analog inhibitors of protein synthesis include *O*-demethylpuromycin, O-propargyl-puromycin, 9-{3'-deoxy-3'-[(4-methyl-L-phenylalanyl)amino]-β-D-ribofuranosyl}-6-(*N,N*'-dimethylamino)purine[L-(4-Me)-Phe-PANS] and 6-dimethylamino-9-[3-(p-azido-L-beta-phenylalanylamino)-3-deoxy-beta-ribofuranosyl] purine. In some embodiments, the polynucleotide linker comprising a polynucleotide (e.g., DNA, RNA or a combination thereof) is linked. In other embodiments, the PEG linker is directly linked to puromycin.

As puromycin that is linked to the 3' terminal end of a RNA molecule enters a ribosome, it establishes a covalent bond to the nascent protein (encoded by the RNA molecule) as a result of peptidyl transferase activity in the ribosome. In turn, a stable amide linkage forms between the protein and the *O*-methyl tyrosine portion of puromycin.

The RNA library of RNA-puromycin fusions can undergo *in vitro* translation, as described herein, to generate RNA-puromycin-protein complexes (*e.g*., RNA-puromycin-Fab fragment complexes). The RNA-puromycin-Fab fragment complexes contain RNA molecules encoding V_{H} chains.

Affinity selection can be performed on the mRNA-displayed Fab fragment library to screen for Fab fragments that specifically bind to a target antigen. Methods described herein, *e.g*., in solution or on a solid support can be used. The selected mRNA-displayed Fab fragments can be purified by standard methods known in the art such as affinity chromatography. The mRNA can be cloned, PCR amplified, and/or sequenced to determine the coding sequence of the heavy chain or light chain of the selected Fab fragment. In one embodiment, the selected mRNA-displayed library contains a population of V_{H} chains.

In some aspects of the invention, members of first library are linked to puromycin or a derivative thereof. In some embodiments, step (iv) of the method includes translating the RNA of the first library in a cell free protein synthesis system to generate a mRNA display system. The mRNA display system can contain a population of complexes such that each complex has a RNA-puromycin molecule and a V_{H} chain. In some embodiments, step (iv) includes generating a library of Fab fragment members wherein library members comprise a complex of a V_{H} chain, in association with a V_{L} chain where the V_{H} chain is associated with a RNA-puromycin molecule.

### B. cDNA display

Similar to mRNA display, cDNA display technology uses a puromycin- polynucleotide linker to generate a RNA/cDNA-protein fusion molecule that can be screened for affinity selection. As described in Yamaguchi et al. (Nucleic Acids Research, 37(16):e108 2009), the system uses puromycin-cDNA linker that can be ligated to an RNA molecule encoding the protein of interest (V_{H} chains), thereby generating an RNA-puromycin-cDNA linker complex.

The complex can undergo cell-free translation to generate a protein-RNA-puromycin-cDNA linker complex. In some embodiments, the population of complexes from the first library contain a strand of an RNA molecule, a puromycin-DNA linker, a ribosome, and a V_{H} chain domain encoded by the RNA. In some embodiments, step (iv) includes generating a library of Fab fragment members wherein library members comprise a complex of a V_{H} chain, in association with a V_{L} chain where the V_{H} chain is associated with a RNA-puromycin-cDNA linker molecule.

Affinity selection can be performed on the cDNA-displayed Fab fragment library to screen for Fab fragments that specifically bind to a target antigen. Methods described herein, *e.g.,* in solution or on a solid support can be used. The selected cDNA-displayed Fab fragments can be purified by standard methods known in the art such as affinity chromatography. The cDNA can be cloned, PCR amplified, and/or sequenced to determine the coding sequence of the heavy chain or light chain of the selected Fab fragment. In one embodiment, the selected cDNA-displayed library contains a population of V_{H} chains.

### C. P2A DNA display

In P2A DNA display, which utilizes the *cis*-activity of the endonuclease P2A, a fusion protein of P2A and an antibody or a fragment thereof (*e.g.,* V_{H} chains) binds to the same DNA molecule from which they are expressed (*see, e.g.,* Reiersen et al., Nucleic Acids Research, 33(1): e10). The DNA template can contain the coding sequence encoding the protein of interest which is genetically fused to the coding sequence of P2A, a promoter, and an origin of replication.

The coding sequence of P2A can be obtained and a genetic fusion of P2A and a V_{H} chain can be constructed by standard methods known to those in the art.

In some embodiments, the library of Fab fragment complexes contains a P2A-Fab fragment fusion protein and a DNA molecule encoding the fusion protein. For example, each member of the library can be associated with a complex comprising a P2A protein which is covalently attached to the 5' phosphate of the DNA encoding it, which is genetically fused to DNA of the first library of the present invention.

The P2A DNA-displayed Fab fragment library can be screened by affinity selection strategies, *e.g*., in solution or on a solid support. The selected Fab fragment can be purified by, *e.g.,* affinity chromatography, and the complexed DNA can be PCR amplified, cloned and/or sequenced.

### D. CIS display

Similar to P2A DNA display, CIS display involves a DNA-based approach to directly link *in vitro* transcribed/translated proteins (*e.g.,* V_{H} chains) to the DNA molecules that encode them (*see, e.g.,* Odegrip et al., Proc. Natl. Acad. Sci. USA, 101(9):2806-2810). This method uses RepA, a DNA replication initiator protein, to non-covalently bind to the DNA molecule from which it is expressed if the DNA molecule has a *CIS* element. The DNA molecule can be created to encode proteins of interest (*e.g*., V_{H} chains) in addition to RepA. It can be contemplated that a functional equivalent of Rep A can be used as a substitute for RepA in the method provided herein.

In some embodiments of the method, members of the DNA library contain a coding sequence of a V_{H} chain, coding sequence of RepA, *CIS* element, origin of replication and a promoter, wherein the coding sequence of the V_{H} chain is genetically fused to the coding sequence of RepA. The *CIS* element can be genetically linked to the RepA coding sequence.

In some embodiments, the library of Fab fragment complexes contains a RepA-Fab fragment fusion protein and a DNA molecule encoding the fusion protein.

The RepA DNA-displayed Fab fragment library can be screened by affinity selection strategies, *e.g*., in solution or on a solid support. The selected Fab fragment can be purified by, *e.g.,* affinity chromatography, and the complexed DNA can be PCR amplified, cloned and/or sequenced.

### VII. Cell-free protein synthesis (CFPS)

Cell extracts used in cell-free protein synthesis have been developed that support the synthesis of proteins *in vitro* from purified mRNA transcripts or from mRNA transcribed from DNA during the *in vitro* synthesis reaction. In some embodiments, the reaction mix of CFPS comprises bacterial extracts and/or defined reagents.

The term "reaction mix" of the CFPS system refers to a reaction mixture capable of catalyzing the synthesis of polypeptides from a nucleic acid template. The reaction mixture comprises extracts from bacterial cells, *e.g*, *E. coli* S30 extracts. The reaction mix of the cell free protein system comprises at least ATP or an energy source; a template for production of the macromolecule, *e.g.,* DNA, mRNA, *etc*.; amino acids, and such co-factors, enzymes and other reagents that are necessary for polypeptide synthesis, *e.g*., ribosomes, tRNA, polymerases, transcriptional factors, aminoacyl synthetases, elongation factors, initiation factors, *etc*. In one embodiment, the energy source is a homeostatic energy source. Also included may be enzyme(s) that catalyze the regeneration of ATP from high-energy phosphate bonds, *e.g*., acetate kinase, creatine kinase, *etc.* Such enzymes may be present in the extracts used for translation, or may be added to the reaction mix. Such synthetic reaction systems are well-known in the art, and have been described in the literature.

CFPS systems can also be engineered to guide the incorporation of detectably labeled amino acids, or unconventional or unnatural amino acids, into a desired protein. The amino acids can be synthetic or derived from another biological source. Various kinds of unnatural amino acids, including without limitation detectably labeled amino acids, can be added to CFPS reactions and efficiently incorporated into proteins for specific purposes. *See,* for example, Albayrak, C. and Swartz, JR., Biochem. Biophys Res. Commun., 431(2):291-5; Yang WC et al., Biotechnol. Prog. (2012), 28(2):413-20; Kuechenreuther et al., PLoS One, (2012), 7(9):e45850; and Swartz JR., AIChE i, 58(1):5-13.

In a generic CFPS system, a coding sequence encoding a protein of interest is expressed in a transcription buffer, resulting in mRNA that is translated into the protein of interest in a CFPS extract and a translation buffer. The transcription buffer, cell-free extract and translation buffer can be added separately, or two or more of these solutions can be combined before their addition, or added contemporaneously. In some instances, the CFPS system is a cell-free translation system wherein the coding sequence is an RNA template of the protein of interest.

To synthesize a protein of interest *in vitro,* a CFPS extract at some point comprises a mRNA molecule that encodes the protein of interest. In some CFPS systems, mRNA is added exogenously after being purified from natural sources or prepared synthetically *in vitro* from cloned DNA using RNA polymerases such as RNA polymerase II, SP6 RNA polymerase, T3 RNA polymerase, T7 RNA polymerase, RNA polymerase III and/or phage derived RNA polymerases. In other systems, the mRNA is produced *in vitro* from a template DNA; both transcription and translation occur in this type of CFPS reaction. In other systems, transcription and translation systems are coupled or complementary transcription and translation systems, which carry out the synthesis of both RNA and protein in the same reaction, have been developed. In such *in vitro* transcription and translation systems, the CFPS extracts contain all the components (exogenous or endogenous) necessary both for transcription (to produce mRNA) and for translation (to synthesize protein) in a single system.

In some embodiments, the CFPS reaction mixture comprises the following associated cofactors: a template nucleic acid, such as DNA, that comprises a coding sequence (gene) of interest operably linked to at least one promoter and, optionally, one or more other regulatory sequences (*e.g*., a cloning or expression vector containing the gene of interest) or a PCR fragment; an RNA polymerase that recognizes the promoter(s) to which the gene of interest is operably linked and, optionally, one or more transcription factors directed to an optional regulatory sequence to which the template nucleic acid is operably linked; ribonucleotide triphosphates (rNTPs); optionally, other transcription factors and co-factors thereof; ribosomes; transfer **RNA** (tRNA); other or optional translation factors (*e.g*., translation initiation, elongation and termination factors) and co-factors therefore; one or more energy sources, (*e.g*., ATP, GTP); optionally, one or more energy regenerating components (*e.g*., PEP/pyruvate kinase, AP/acetate kinase or creatine phosphate/creatine kinase); optionally factors that enhance yield and/or efficiency (*e.g*., nucleases, nuclease inhibitors, protein stabilizers, chaperones) and co-factors thereof; and; optionally, solubilizing agents. The reaction mix can further comprises amino acids and other materials specifically required for protein synthesis, including salts (*e.g*., potassium, magnesium, ammonium, and manganese salts of acetic acid, glutamic acid, or sulfuric acids), polymeric compounds (*e.g*., polyethylene glycol, dextran, diethyl aminoethyl dextran, quaternary aminoethyl and aminoethyl dextran, etc.), cyclic AMP, inhibitors of protein or nucleic acid degrading enzymes, inhibitors or regulators of protein synthesis, oxidation/reduction adjuster (*e.g*., DTT, ascorbic acid, glutathione, and/or their oxides), non-denaturing surfactants (*e.g.,* Triton X-100), buffer components, spermine, spermidine, putrescine, *etc.* Components of CFPS reactions are discussed in more detail in U.S. Patent Nos. 7,338,789 and 7,351,563, and U.S. App. Pub. No. 2010/0184135.

Depending on the specific enzymes present in the bacterial extract, for example, one or more of the many known nuclease, polymerase or phosphatase inhibitors can be selected and advantageously used to improve synthesis efficiency.

Protein and nucleic acid synthesis typically requires an energy source. Energy is required for initiation of transcription to produce mRNA (*e.g*., when a DNA template is used and for initiation of translation high energy phosphate for example in the form of GTP is used). Each subsequent step of one codon by the ribosome (three nucleotides; one amino acid) requires hydrolysis of an additional GTP to GDP. ATP is also typically required. For an amino acid to be polymerized during protein synthesis, it must first be activated. Significant quantities of energy from high energy phosphate bonds are thus required for protein and/or nucleic acid synthesis to proceed.

An energy source is a chemical substrate that can be enzymatically processed to provide energy to achieve desired chemical reactions. Energy sources that allow release of energy for synthesis by cleavage of high-energy phosphate bonds such as those found in nucleoside triphosphates, *e.g*., ATP, are commonly used. Any source convertible to high energy phosphate bonds is especially suitable. ATP, GTP, and other triphosphates can normally be considered as equivalent energy sources for supporting protein synthesis.

To provide energy for the synthesis reaction, the system preferably includes added energy sources, such as glucose, pyruvate, phosphoenolpyruvate (PEP), carbamoyl phosphate, acetyl phosphate, creatine phosphate, phosphopyruvate, glyceraldehyde-3-phosphate, 3-Phosphoglycerate and glucose-6-phosphate, that can generate or regenerate high-energy triphosphate compounds such as ATP, GTP, other NTPs, etc.

When sufficient energy is not initially present in the synthesis system, an additional source of energy is preferably supplemented. Energy sources can also be added or supplemented during the *in vitro* synthesis reaction.

In some embodiments, the CFPS reaction is performed using the PANOx-SP system comprising NTPs, *E.coli* tRNA, amino acids, Mg²⁺ acetate, Mg²⁺ glutamate, K⁺ acetate, K⁺ glutamate, folinic acid, Tris pH 8.2, DTT, pyruvate kinase, T7 RNA polymerase, disulfide isomerase, phosphoenol pyruvate (PEP), NAD, CoA, Na⁺ oxalate, putrescine, spermidine, and S30 extract.

In some instances, the CFPS reaction does not require the addition of commonly secondary energy sources, yet uses co-activation of oxidative phosphorylation and protein synthesis. In some instances, CFPS is performed in a reaction such as the Cytomim^{™} (cytoplasm mimic) system. The Cytomim^{™} system is defined as a reaction condition performed in the absence of polyethylene glycol with optimized magnesium concentration. This system does not accumulate phosphate, which is known to inhibit protein system.

In some embodiments, the CFPS reaction is performed using the Cytomim^{™} system comprising NTPs, *E.coli* tRNA, amino acids, Mg²⁺ acetate, Mg²⁺ glutamate, K⁺ acetate, K⁺ glutamate, folinic acid, Tris pH 8.2, DTT, pyruvate kinase, T7 RNA polymerase, disulfide isomerase, sodium pyruvate, NAD, CoA, Na⁺ oxalate, putrescine, spermidine, and S30 extract. In some embodiments, the energy substrate for the Cytomim system is pyruvate, glutamic acid, and/or glucose. In some embodiments of the system, the nucleoside triphosphates (NTPs) are replaced with nucleoside monophosphates (NMPs).

The cell extract of the reaction mix can be treated with iodoacetamide in order to inactivate enzymes that can reduce disulfide bonds and impair proper protein folding. The cell extract can also be treated with a prokaryotic disulfide bond isomerase, such as, not limited to, *E*. *coli* DsbC and PDI. The cell extract can be treated with DsbC in addition to FkpA and peptidyl peolyl isomerase. Glutathione disulfide (GSSG) and glutathione (GSH) can also be added to the extract at a ratio that promotes proper protein folding and prevents the formation of aberrant protein disulfides. In some embodiments, molecular chaperones such as DnaK, DnaJ, GrpE, GroEL or GroES can be added to the CFPS reaction.

In some embodiments, the CFPS reaction includes inverted membrane vesicles to perform oxidative phosphorylation to produce ATP. These vesicles can be formed during the high pressure homogenization step of the preparation of cell extract process, and remain in the extract used in the reaction mix of CFPS.

In some instances, the CFPS reaction includes a component that can block the 10s-RNA system which can tag RNA transcripts without a stop codon. In some embodiments, the CFPS reaction is free of ssrA-RNA. For example, the bacterial extract of the reaction can be derived from an *E.coli* strain lacking a function ssrA gene such as X90 ssrA1::cat (Keiler et al., Science 221:1990-1993 (1996)), N2211 or NM101 (Tu et al., J. Biol. Chem. 270:9322-9326 (1995)), W3110 ΔssrA (Komine et al., Proc. Natl. Acad. Sci. U.S.A. 91:9223-9227 (1994)), and K7823 or K6676 (Retallack and Friedman, Cell 83:227-235 (1995)). In some embodiments, an antisense ssrA oligonucleotide or other inhibitory RNA directed against ssrA can be added to the CFPS reaction.

Methods of preparing a cell extract are described in, *e.g*., Zawada, J. "Preparation and Testing of E.coli S30 In Vitro Transcription Translation Extracts", Douthwaite, J.A. and Jackson, R.H. (eds.), Ribosome Display and Related Technologies: Methods and Protocols, Methods in Molecular Biology, vol. 805, pp. 31-41 (Humana Press, 2012); Jewett et al., Molecular Systems Biology: 4, 1-10 (2008); Shin J. and Norieaux V., J. Biol. Eng., 4:8 (2010). Briefly, a bacterial culture is grown and harvested; suspended in an appropriate buffer *(e.g.,* S30 buffer), and homogenized to lyse the cells.

An embodiment uses a bacterial cell from which a lysate is derived. A bacterial lysate derived from any strain of bacteria can be used in the methods of the invention. The bacterial lysate can be obtained as follows. The bacteria of choice are grown up to log phase in any of a number of growth media and under growth conditions that are well known in the art and easily optimized by a practitioner for growth of the particular bacteria. For example, a natural environment for synthesis utilizes cell lysates derived from bacterial cells grown in medium containing glucose and phosphate, where the glucose is present at a concentration of at least about 0.25% (weight/volume), more usually at least about 1%; and usually not more than about 4%, more usually not more than about 2%. An example of such media is 2YTPG medium, however one of skill in the art will appreciate that many culture media can be adapted for this purpose, as there are many published media suitable for the growth of bacteria such as E. coli, using both defined and undefined sources of nutrients. Cells that have been harvested overnight can be lysed by suspending the cell pellet in a suitable cell suspension buffer, and disrupting the suspended cells by sonication, breaking the suspended cells in a French press, continuous flow high pressure homogenization, or any other method known in the art useful for efficient cell lysis. The cell lysate is then centrifuged or filtered to remove large DNA fragments and cell debris.

The bacterial strain used to make the cell lysate generally has reduced nuclease and/or phosphatase activity to increase cell free synthesis efficiency. For example, the bacterial strain used to make the cell free extract can have mutations in the genes encoding the nucleases RNase E and RNase A. The strain may also have mutations to stabilize components of the cell synthesis reaction such as deletions in genes such as *tnaA, speA, sdaA or gshA,* which prevent degradation of the amino acids tryptophan, arginine, serine and cysteine, respectively, in a cell-free synthesis reaction. Additionally, the strain may have mutations to stabilize the protein products of cell-free synthesis such as knockouts in the proteases ompT or lonP.

In some embodiments, the bacteria extract can be thawed to room temperature before use in the CFPS reaction. The extract can be incubated with 50 µM iodoacetamide for 30 minutes when synthesizing protein with disulfide bonds. In some embodiments, the CFPS reaction includes about 30% (v/v) iodoacetamide-treated extract with about 8 mM magnesium glutamate, about 10 mM ammonium glutamate, about 130 mM potassium glutamate, about 35 mM sodium pyruvate, about 1.2 mM AMP, about 0.86 mM each of GMP, UMP, and CMP, about 2 mM amino acids (about 1 mM for tyrosine), about 4 mM sodium oxalate, about 0.5 mM putrescine, about 1.5 mM spermidine, about 16.7 mM potassium phosphate, about 100 mM T7 RNA polymerase, about 2-10 µg/mL plasmid DNA template, about 1-10 µM E.coli DsbC, and a total concentration of about 2 mM oxidized (GSSG) glutathione. Optionally, the cell free extract can include 1 mM of reduced (GSH) glutathione.

### VIII. Screening methods of the library for selection of affinity to target antigen

Screening methods of the Fab complex can be performed with a target antigen immobilized on a surface or in solution. Immobilized antigen is affixed to a solid phase substrate such as a well, bead, particle, plate, glass slide, microarray, or chip. In some instances, the immobilized antigen is bound to the surface of a cell.

The binding affinity of a Fab fragment to a target antigen can be determined to select the Fab fragment that has high affinity for the antigen. Fab fragments can be selected that have a binding affinity that is or is about 10⁻⁶ M, 10⁻⁷ M, 10⁻⁸ M, 10⁻⁹ M, 10⁻¹⁰ M, 10⁻¹¹ M, 10⁻¹² M or less. If the Fab fragments do not have the desired binding affinity in the first round of screening (capturing), the Fab fragments can be optimized by iterative screening and/or directed evolution methods (e.g., random mutagenesis) and further screened for binding to a target antigen to identify a Fab fragment that has a high binding affinity.

### IX. Iterative screening for evolution or enrichment of antibody

If the Fab fragments do not have the desired binding affinity after screening, the Fab fragments can be optimized by iterative screening and/or directed evolution methods (e.g., random mutagenesis) and further screened for binding to the target antigen to identify a Fab fragment that has a high binding affinity.

The RNA translated by the ribosome of the selected Fab fragment complex can be reverse transcribed into single stranded cDNA, converted to double-stranded DNA (dsDNA) and then, amplified by PCR. The reverse transcription reaction can be performed on mRNA associated in the ribosomal display complex, or the mRNA can be isolated from the ribosomal display complex then used in the reverse transcription step. This method can be used to obtain the coding sequence of the selected variable chain domain of the first library.

As the first DNA library encodes V_{H} variable chain domains, the RNA of the V_{H} chain of the selected Fab fragment can be reverse transcribed into cDNA and made into dsDNA. The DNA can be sequenced and/or amplified by PCR. The DNA can also be mutated by methods such as site-directed mutagenesis, error-prone PCR, homologous recombination, DNA shuffling, etc., to increase the diversity of the variable chain for another round of screening. In some embodiments, another first library based on the selected V_{H} chain is generated and combined with the second library of V_{L} chain member to generate another library of Fab fragment members. The Fab fragment library can be screening and selected for binding to the target antigen.

### X. Examples

### Example 1. Fab heavy chain and light chain display

This example illustrates a method for selecting a heterodimeric Fab domain by ribosome display using two templates: (1) a display template containing the Fab heavy chain (HC) fused to a TolA spacer without a stop codon, and (2) a complementary expression template containing the Fab light chain (LC) with a stop codon (FIG. 1A). The lack of a stop codon at the end of the display template forces stalling of the ribosome at the end of the HC mRNA transcript enabling formation of the ternary protein-ribosome-mRNA complex. The TolA spacer is a standard unstructured domain used in ribosome display which allows the nascent Fab HC to project from the exit tunnel and fold outside of the ribosome. The LC expression template contains a stop codon at the end of the transcript and lacks the TolA spacer since it is not intended to be displayed directly from the ribosome nor linked to its own mRNA transcript. Instead, co-expression of the LC enables heterodimerization with the displayed HC on the ribosome to yield the complete Fab-ribosome-HC mRNA ternary complex (FIG. 1B). Alternatively, but not part of the invention, the Fab can be linked to the LC mRNA by expressing the LC from the display template and its complementary HC from an expression template. In this manner, libraries of either the HC or LC can be screened in turn.

The HC display and expression templates were made by PCR of the Herceptin HC Fab plasmid using either the T7B/TolAk primers or T7B/HER2_FAB_HC_STOP! RP (Table 1), respectively. The LC display and expression templates were made by PCR of the Herceptin Fab LC plasmid using either the T7B/TolAk primers, or the T7B/Her2_FAB_LC_STOP! RP, respectively. All DNA templates were then purified with a Qiagen PCR purification kit. A total of 5 µL of a 3:1 mixture (based on mass) of the appropriate expression and display templates were added to 20 µL of pre-mixed PURExpress *in vitro* protein synthesis kit (NEB) containing 10 µL solution A, 7.5 µL solution B, 1 µL disulfide enhancer 1, 1µL disulfide enhancer 2, 0.5 µL nuclease-free water (Ambion). The final concentration of the expression and display DNA templates were 300 ng/µL and 100 ng/µL, respectively. The coupled transcription/translation reactions were allowed to proceed for 60 min at 30 °C. The remaining selection was performed using standard protocols (Dreier and Plückthun, Methods Mol. Biol. 2011 687, 283-306) with 14 nM HER2/ErbB2 ECD (Creative Biomart) previously biotinylated. RT-PCR was performed using a 1-step Transcriptor RT-PCR kit (Roche) with T7B/TolAk primers (FIG. 1C). The RT-PCR product was sequenced using the TolA Internal RP which verified recovery of the displayed HC or LC protein fragment. These experiments suggest functional heterodimeric Fab domains can be displayed from the ribosome with either the HC or LC mRNA connected to its respective HC or LC protein.

**Table 1. Primer for PCR and RT-PCR*.**

| ***Name*** | ***Sequence (5' to 3')*** | |
|---|---|---|
| HER2_FAB_HC_STOP! RP | TTA TTA ACA ACA AGA TTT CGG CTC CAC CT | SEQ ID NO: 15 |
| HER2_FAB_LC_STOP! RP | TTA TTA GCA CTC ACC GCG GTT AAA | SEQ ID NO: 16 |
| FAB FP | ATG GAA GTT CAA TTA GTA GAA AGC GGC G | SEQ ID NO: 17 |
| FAB RP | ACA AGA TTT CGG CTC CAC CTT CTT G | SEQ ID NO: 18 |
| Fc FP | CAA GAA GGT GGA GCC GAA ATC TTG T | SEQ ID NO: 19 |
| Fc RP | CTT ACC CGG GGA CAG GGA CAA G | SEQ ID NO: 20 |
| HC FP | | SEQ ID NO: 21 |
| HC RP | TTT TGT CGA CCT TAC CCG GGG ACA GGG ACA AG | SEQ ID NO: 22 |
| Univ TolA RP | TTC AGT TGC CGC TTT CTT TC | SEQ ID NO: 23 |
| HER2 HCFAB Inner FP | | SEQ ID NO: 24 |
| HER2 HCFAB Inner RP | GAG GCG ATA TAA AGC TTA CAA GAT TTC GG | SEQ ID NO: 25 |
| TolA Internal RP | CTT TGG CGG CTT CTG CTT | SEQ ID NO: 26 |
| * T7B and TolAk primers have been previously reported by Dreier and Plückthun, Methods Mol. Biol. 687, 283-306, (2011). | | |

In summary, this example illustrates that the formation of a ternary complex of the functional Fab, ribosome, mRNA complex using a mixture of a display template and its complementary expression template co-expressed in a cell-free translation mixture. For HC display, the HC display template was co-expressed with the LC expression template which enables selection of a HC library. Alternatively, co-expression of the LC display template with the HC expression template enabled LC display. In particular, the example shows that co-expression of both the Herceptin Fab HC and LC display and expression templates in either the HC or LC display format is required for significant recovery of the template following selection with HER2/ErbB2 extra-cellular domain (ECD). These results are consistent with the notion that functional heterodimeric Fabs can be assembled on stalled ribosomes.

This work has been published (see, Stafford et al., Protein Eng Des Sel, 27(4):97-109 (2014)) which is incorporated herein by reference in its entirety.

### Example 2. DNA library design for Fab fragments

The Fab HC library was built onto the trastuzumab Fab variable heavy chain (HC) scaffold. The trastuzumab scaffold was cloned into the NcoI and HindIIIl sites of a pUC ribosome display vector (pRDV) containing the TolA spacer (GenBank Accession No. AY327136.1). The Fab HC only library design was based on the published library (Lee et al., JMB, 340:1073-1093 (2004)) using the same limited diversity in CDRs H1 and H2. CDR H3 was restricted to a single loop length of 11 amino acids and 8 residues were randomized using NNK codons. The library was constructed using overlapping PCR as described below.

For the Fab HC DNA library, synthetic oligonucleotides (Table 2) containing degenerate codons were dissolved to generate 100 µM stocks. T7B and TolAk primers have been previously reported (Dreier and Plückthun, Methods Mol. Biol., 687: 283-306 (2011)). Primer sets were mixed to give similar degeneracy as Lee *et al.* H1a For and H1b For primers were mixed at a 2:1 ratio to make a H1 For primer mix. H2a For, H2b For, and H2c For primers were mixed at a ratio of 1:2:0.1 to give a H2 For primer mix. H3a For and H3a For primers were mixed at a ratio of 1:1 to give H3 For primer mix. The complementary reverse primer sets were mixed separately at the same ratios. Four sets of PCR reactions were performed using (1) T7B/H1 Rev mix, (2) H1 For mix/H2 Rev mix, (3) H2 For mix/H3 Rev mix, and (4) H3F mix and Univ TolA RP. PCR was performed using 10 ng of the wild-type Fab HC ribosome display template and 0.5 µL of 100 µM primer stocks (about 3 × 10¹³ molecules) using 2x HF Phusion polymerase master mix (NEB). The fragments were gel purified and mixed at equimolar ratios (about 3.7 × 10¹² molecules each) for overlapping PCR (18 × 50 µL reactions). The assembled library was then amplified using T7B/TolAk primers (48 × 50 µL reactions) which was then purified and concentrated using Zymo Research DNA Clean and Concentrator spin columns.

**Table 2. Primers for Fab variable heavy chain library construction**

| | Primer Name | Sequence (5' to 3') |
|---|---|---|
| SEQ ID NO: 1 | H1a For | |
| SEQ ID NO: 2 | H1a Rev | |
| SEQ ID NO: 3 | H1b For | |
| SEQ ID NO: 4 | H1b Rev | |
| SEQ ID NO: 5 | H2a For | |
| SEQ ID NO: 6 | H2a Rev | |
| SEQ ID NO: 7 | H2b For | |
| SEQ ID NO: 8 | H2b Rev | |
| SEQ ID NO: 9 | H2c For | |
| SEQ ID NO: 10 | H2c Rev | |

| (continued) | | |
|---|---|---|
| | Primer Name | Sequence (5' to 3') |
| SEQ ID NO: 11 | H3a For | |
| SEQ ID NO: 12 | H3a Rev | |
| SEQ ID NO: 13 | H3b For | |
| SEQ ID NO: 14 | H3b Rev | |

### Example 3. Identifying Fab fragments using ribosomal display and the PURE system

### A. Selection of Fab fragments in PURE system

This example illustrates the method of selecting Fab fragments from a Fab library, wherein the heavy chain library is generated from a DNA library and a reconstituted ribosome system (*e.g*., PURE system). The method includes selecting Fab fragments that specifically bind to a target antigen by using ribosomal display.

An aliquot of the Fab HC library was purified by gel extraction from a 1% agarose gel before the selection. The first round of selection was performing using coupled transcription/translation reactions (10 × 25 µL each) were each performed using 7.5 µg pre-purified variable light chain protein and 248 ng of the DNA library. For subsequent rounds, only 2 reactions were performed for the plus target antigen set (+Ag; 25 µL each) and 2 reactions for the negative target antigen set (-Ag; 25 µL each) using between 130-230 ng DNA for each reaction.

Coupled transcription/translation were run adding 5 µL of the purified library to 20 µL of pre-mixed PURExpress *in vitro* protein synthesis kit (New England Biolabs, Ipswich, MA) containing 10 µL solution A, 7.5 µL solution B, 1 µL disulfide enhancer 1, 1 µL disulfide enhancer 2. The reactions were allowed to proceed for 60 min at 30 °C before quenching. Fabs specific for the target antigen were isolated using biotinylated antigen (biotinylated using standard protocol).

The target antigen was biotinylated with EZ Link NHS-PEO₄ (Cat. #21329, Thermo Fisher Scientific, Waltham, MA) resulting in approximately one biotin molecule per antigen molecule as determined with the Fluorescence Biotin Quantitation Kit (CAT. #46610, Thermo Fisher, Waltham, MA).

RT-PCR was performed to isolate the coding sequence of selected Fabs using a 1-step Transcriptor RT-PCR kit (Roche) with primers specific to the trastuzumab Fab HC library scaffold. The remaining selection was performed using standard protocols (Dreier and Plückthun, Methods Mol. Biol. 2011 687, 283-306).

### B. Characterization of IgGs converted from Fabs selected in the PURE system

This section describes the generation of full-length IgGs from Fab fragments selected by ribosomal display as described in the previous section. It also provides methods for analyzing the IgGs selected for binding to a target antigen such as CEA.

Cell-free reactions were run essentially as described previously using DsbC, PDI, and pre-purified Herceptin LC protein (Yin et al., mAbs, 4:217-225 (2012)). Small-scale coupled transcription/translation was initiated by addition of 5 µL of non-normalized mini-prepped DNA (approximately 50-150 ng/µL) to the cell-free mixture (30 µL final volume) and was incubated at 30 °C for 6 hours in a covered 96-well microtiter plate while shaking at 800 rpm using an Eppendorf Thermomixer R. The crude cell-free reactions were stored at 4 °C until they were centrifuged at 5,000g for 5 min at 4 °C. The supernatant was diluted into 500 µL PBST.

A separate MaxiSorp plate was incubated overnight at 4 °C with 100 µL of 5 µg/mL of CEA antigen, HER2/ErbB2 ECD, or streptavidin in PBS. The antigen solution was removed and the plate was blocked with 2% BSA in PBST for 1 hour at room temperature while shaking (500 rpm). Then 100 µL of the diluted crude cell-free reactions were added to the wells and incubated at room temperate for 1 hour with shaking. The plate was washed 5 times with PBST and 100 µL of 1:10,000 dilution of anti-human IgG (Fc specific)-peroxidase antibody conjugate (Sigma) in PBST was incubated for 1 hour at room temperature with shaking. The plate was washed 5 times with PBST and developed with 100 µL TMB for 16 min before quenching with 100 µL 1 N sulfuric acid. Absorbances were measured using a SpectraMax plate reader.

Selected Fab fragments were reformatted (converted) to full-length IgGs by standard method known to those skilled in the art as described in, for example, Palys et al., Hum. Antibodies, 15(4):125-132 (2006) and Guttieri et al., Hybrid Hybridomics, 22(3):135-145.

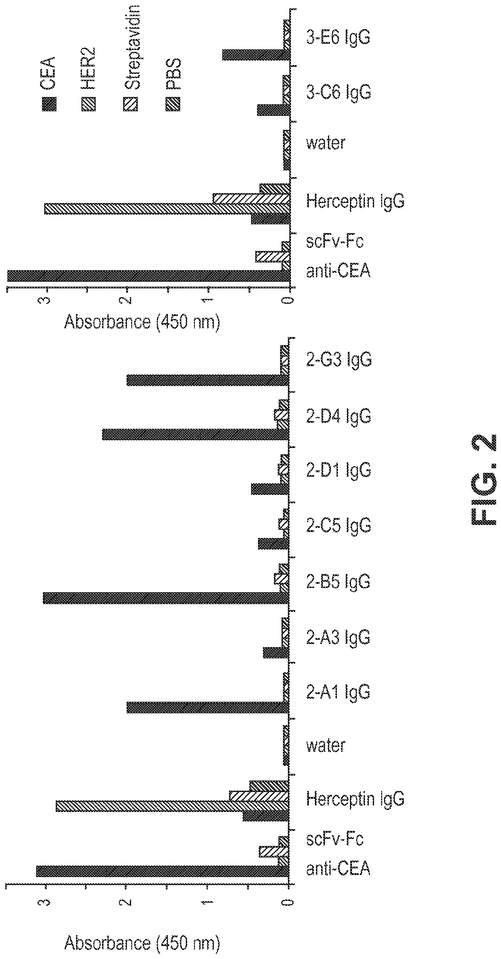

Analysis by ELISA showed that the IgGs reformatted from the selected Fabs can specifically bind to the target antigen CEA (FIG. 2). The size of the reformatted IgGs was assessed by SDS-PAGE (FIG. 3A). The final purified anti-CEA IgGs ran as a single band on a non-reducing SDS-Page gel which is consistant with full-length IgG polypeptides. Under reducing conditions, bands consistent in size with the variable heavy chain and variable light chain fragments were observed. The purity of the selected anti-CEA IgGs was determined by a lab-on-a chip protein assay (Caliper Life Sciences, Hopkinton, MA). The data indicates that the IgGs had high purity (FIG. 3B). Specificity of the selected anti-CEA IgGs was analyzed by ELISA (FIG. 3C). The IgGs had sub-nM EC₅₀ values for CEA. Antibody-antigen interactions and binding affinity was assessed by Biacore technology (GE Healthcare Biosciences, Pittburgh, PA). FIG. 3D shows representative Biacore traces for the 2-G3 anti-CEA IgG (dotted lines) and global fit to a bivalent binding model (black lines). Binding was measured in duplicate at 1.3, 2.5, 5.1, 10.1, 20.3, and 40.5 nM over three separate channels. It was determined that a bivalent binding model which is expected for a bivalent IgG fit the data better than simple 1:1 binding. The IgGs reformatted from the selected Fabs showed high affinities on CEA-expressing MKN45 cells, while wild-type trastuzumab (Herceptin) did not (FIG. 3E). Wild-type trastuzumab specifically bound to HER2-expressing SKBR3 cells, yet the selected anti-CEA IgGs did not (FIG. 3F).

### Example 4. Identifying Fab fragments using ribosomal display and bacterial extracts of the cell-free protein synthesis system

### A. Selection of Fab fragments in bacterial extracts

This example illustrates the method of selecting Fab fragments from a Fab library, wherein the heavy chain library is generated from a DNA library. The method includes generating heavy chains and selecting the Fab fragments that specifically bind to a target antigen. In particular, a cell-free protein synthesis system is used with ribosomal display.

An aliquot of the Fab variable heavy chain library, as described in Example 1, was purified by gel extraction from a 1% agarose gel before the selection. The first round of selection by ribosomal display was performing using coupled transcription/translation reactions (8 × 25 µL each) were each performed using 7.7 µg pre-purified LC protein and about 240 ng of the DNA library (about 2000 ng total or 1.7 x 10¹² molecules). For subsequent rounds only 2 reactions were performed for the plus target antigen set (+Ag; 25 µL each) and 2 reactions for the minus target antigen set (-Ag; 25 µL each) using between 130-230 ng DNA for each reaction. Coupled transcription/translation reactions were performed by adding 3 µL of the purified library to 22 µL of cell-free mix supplemented with pre-purified light chain protein, DsbC, PDI, GSSG, GamS, and an antisense ssrA oligo (Hanes and Pluckthun,Proc. Natl. Acad. Sci. U.S.A, 94, 4937-4942 (1997), U.S. Patent No. 6,589,741). The reactions were allowed to proceed for 60 min at 30 °C before quenching with a quenching buffer (*e.g*., WBT supplemented with 1% BSA and heparin).

Fabs specific for the target antigen were isolated using biotinylated Ag. Briefly, the target antigen was biotinylated with EZ Link NHS-PEO₄ biotin (Cat. #21329, Thermo Fisher Scientific, Waltham, MA) resulting in approximately one biotin molecule per antigen molecule as determined with the Fluorescence Biotin Quantitation Kit (CAT. #46610, Thermo Fisher , Waltham, MA).

RT-PCR was performed to isolate the coding sequence of Fabs using a 1-step Transcriptor RT-PCR kit (Roche Diagnostics, Indianapolis, IN) with primers specific to the Fab HC library scaffold. The remaining rounds of selection were performed using standard protocols, *e.g.,* selection in plates or selection in solution (Dreier and Plückthun, Methods Mol. Biol. 2011 687, 283-306). Six rounds of selection were performed until the recovery was greater for +Ag versus -Ag, as assessed by RT-PCR.

### B. Characterization of IgGs converted from Fabs selected in bacterial extracts

This section illustrates the generation of full-length IgGs from Fab fragments selected by ribosomal display in a bacterial extract. It also demonstrates methods for analyzing the IgGs selected for binding to a target antigen.

Cell-free reactions were run essentially as described previously using DsbC and PDI (Yin et al. mAbs, 4(2):217-225 (2012)). Small-scale coupled transcription/translation was initiated by addition of 5 µL of non-normalized mini-prepped IgG variable heavy chain DNA (approximately 50-150 ng/µL) and IgG variable light chain DNA to the cell-free mixture (30 µL final volume) and was incubated at 30 °C for 12 hours in a covered 96-well microtiter plate while shaking at 800 rpm using an Eppendorf Thermomixer R. The crude cell-free reactions were stored at 4 °C until they were centrifuged at 5,000g for 5 min at 4 °C. The supernatant was diluted into 500 µL PBST. A separate MaxiSorp plate (Thermo Fisher, Waltham, MA) was incubated overnight at 4 °C with 100 µL of 5 µg/mL of target antigen (Ag) or streptavidin in PBS. The antigen solution was removed and the plate was blocked with 2% BSA in PBST for 1 hour at room temperature while shaking (500 rpm). Then 100 µL of the diluted crude cell-free reactions were added to the wells and incubated at room temperate for 1 hour with shaking. The plate was washed 5 times with PBST and 100 µL of 1:10,000 dilution of anti-human IgG (Fc specific)-peroxidase antibody conjugate (Sigma-Aldrich, St. Louis, MO) in PBST was incubated for 1 hour at room temperature with shaking. The plate was washed 5 times with PBST and developed with 100 µL TMB for 16 min before quenching with 100 µL 1 N sulfuric acid. Absorbances were measured using a SpectraMax plate reader.

Selected Fab fragments were reformatted (converted) to full-length IgGs by standard method known to those skilled in the art as described in, for example, Palys et al., Hum. Antibodies, 15(4):125-132 (2006) and Guttieri et al., Hybrid Hybridomics, 22(3):135-145.

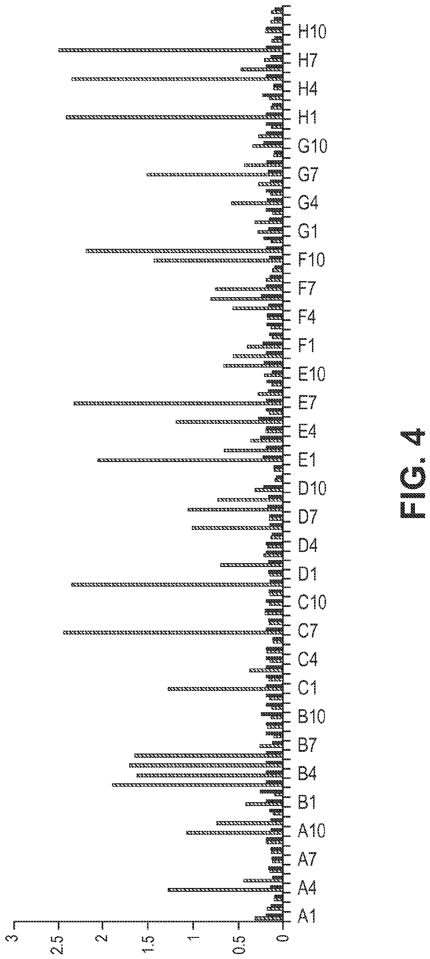

FIG. 4 shows that IgGs reformatted from the Fab fragments selected in ribosomal display specifically bind to the target antigens from which the Fab fragments were selected.

Although the foregoing invention has been described in some detail by way of illustration and example for purposes of clarity of understanding, one of skill in the art will appreciate that certain changes and modifications may be practiced within the scope of the appended claims.

## Claims

1. A method for selecting a Fab fragment of interest from a library of Fab fragments comprising variable heavy (VH) chain domains and variable light (VL) chain domains, the method comprising:
i) generating a first library of DNA encoding V_{H} chain domains where the library comprises at least 10² different library members, each member having a different base sequence;
ii) generating a second library of one V_{L} chain domain member;
iii) transcribing the first library of DNA to RNA;
iv) translating the RNA of the first library in a cell free protein synthesis system to generate a ribosomal display reaction system comprising a population of complexes comprising a strand of an RNA molecule, a ribosome and V_{H} chain domains to generate a library of Fab fragment members where library members comprise a complex comprising a V_{H} chain, in association with a V_{L} chain domain where the VH chain is associated with an RNA molecule and a ribosome, wherein the step of translating the RNA of the first library and generation of the V_{L} chain domains of the second library occur separately such that the generated V_{L} chain domains of the second library are added to the step of translating the RNA of the first library; and v) selecting the Fab fragments of interest from the library of Fab fragments.

2. The method of claim 1, wherein the first library has between 10³ and 10⁵ members or between 10⁵ and 10¹⁶ members.

3. The method of claim 1, wherein the ribosomal display reaction system comprises a puromycin-based display or a CIS display.

4. The method of claim 1, further comprising removing the ribosome of the ribosomal display reaction system prior to step (v) of selecting the Fab fragments of interest.

5. The method of claim 1, wherein the selecting step comprises capturing the Fab fragments with an immobilized antigen.

6. The method of claim 1, wherein the RNAs of the Fab fragments selected from step (v) are amplified and the amplified products are combined with a library and a cell free, ribosomal display reaction system to generate a population of complexes comprising a strand of RNA, a ribosome and Fab fragments that are enriched for binding to the antigen used to capture the Fab fragments of step (v).

7. The method of claim 1, wherein the cell free protein synthesis system comprises
(a) an oxidative phosphorylation reaction producing ATP;
(b) a reconstituted ribosome system; or
(c) a bacterial extract with associated co-factors.

## Patentansprüche

1. Verfahren zum Auswählen eines gewünschten Fab-Fragments aus einer Bibliothek von Fab-Fragmenten, die Domänen der variablen schweren (V_{H}) Kette und Domänen der variablen leichten (V_{L}) Kette umfassen, wobei das Verfahren Folgendes umfasst:
i) Erzeugen einer ersten Bibliothek von DNA-codierenden Domänen der V_{H}-Kette, wobei die Bibliothek mindestens 10² verschiedene Bibliotheksmitglieder umfasst, wobei jedes Mitglied eine unterschiedliche Basensequenz hat;
ii) Erzeugen einer zweiten Bibliothek mit einem Mitglied der Domäne der V_{L}-Kette;
iii) Transkribieren der ersten Bibliothek von DNA zu RNA,
iv) Translation der RNA der ersten Bibliothek in einem zellfreien Proteinsynthesesystem, um ein ribosomales Anzeigereaktionssystem, das eine Population von Komplexen umfasst, die einen Strang eines RNA-Moleküls umfassen, ein Ribosom und Domänen der V_{H}-Ketten zu erzeugen, um eine Bibliothek von Fab-Fragmentmitgliedern zu erzeugen, wobei die Bibliotheksmitglieder einen Komplex umfassen, der eine V_{H}-Kette umfasst, in Verbindung mit einer Domäne der V_{L}-Kette, wobei die V_{H}-Kette mit einem RNA-Molekül und einem Ribosom verbunden ist, wobei der Schritt der Translation der RNA der ersten Bibliothek und die Erzeugung der Domänen der V_{L}-Kette der zweiten Bibliothek getrennt auftreten, sodass die erzeugten Domänen der V_{L}-Kette der zweiten Bibliothek zu dem Schritt der Translation der RNA der ersten Bibliothek hinzugefügt werden; und
v) Auswählen der gewünschten Fab-Fragmente aus der Bibliothek von Fab-Fragmenten.

2. Verfahren nach Anspruch 1, wobei die erste Bibliothek zwischen 10³ und 10⁵ Mitglieder oder zwischen 10⁵ und 10¹⁶ Mitglieder hat.

3. Verfahren nach Anspruch 1, wobei das ribosomale Anzeigereaktionssystem eine puromycinbasierte Anzeige oder eine CIS-Anzeige umfasst.

4. Verfahren nach Anspruch 1, ferner umfassend ein Entfernen des Ribosoms des ribosomalen Anzeigereaktionssystems vor Schritt (v) des Auswählens des gewünschten Fab-Fragments.

5. Verfahren nach Anspruch 1, wobei der Auswählschritt ein Einfangen des Fab-Fragments mit einem immobilisierten Antigen umfasst.

6. Verfahren nach Anspruch 1, wobei die RNAs des aus Schritt (v) ausgewählten Fab-Fragments amplifiziert sind und die amplifizierten Produkte mit einer Bibliothek und einem zellfreien ribosomalen Anzeigereaktionssystem kombiniert sind, um eine Population von Komplexen zu erzeugen, die einen RNA-Strang, ein Ribosom und Fab-Fragmente umfassen, die zum Binden des Antigens angereichert sind, das verwendet wird, um die Fab-Fragmente aus Schritt (v) einzufangen.

7. Verfahren nach Anspruch 1, wobei das zellfreie Proteinsynthesesystem Folgendes umfasst
(a) eine oxidative Phosphorylierungsreaktion, die ATP produziert;
(b) ein rekonstituiertes Ribosomsystem; oder
(c) einen Bakterienextrakt mit verbundenen Cofaktoren.

## Revendications

1. Procédé de sélection d'un fragment Fab d'intérêt provenant d'une bibliothèque de fragments Fab comprenant des domaines variables à chaîne lourde (V_{H}) et des domaines variables à chaîne légère (V_{L}), le procédé comprenant :
i) la génération d'une première bibliothèque d'ADN codant pour des domaines variables à chaîne V_{H} où la bibliothèque comprend au moins 10² différents éléments de bibliothèque, chaque élément comportant une séquence de base différente ;
ii) la génération d'une deuxième bibliothèque d'un élément de domaine à chaîne V_{L} ;
iii) la transcription de la première bibliothèque d'ADN en ARN ;
iv) la traduction de l'ARN de la première bibliothèque en système de synthèse de protéines acellulaires pour générer un système réactionnel d'affichage de ribosomes comprenant une population de complexes comprenant un brin d'une molécule d'ARN, un ribosome et des domaines à chaîne V_{H} pour générer une bibliothèque d'éléments de fragment Fab où des éléments de bibliothèque comprennent un complexe comprenant une chaîne V_{H}, en association avec un domaine à chaîne V_{L} où la chaîne V_{H} est associée à une molécule ARN et un ribosome, l'étape de traduction de l'ARN de la première bibliothèque et la génération des domaines à chaîne V_{L} de la deuxième bibliothèque se produisant séparément de manière que les domaines à chaîne V_{L} de la deuxième bibliothèque sont ajoutés à l'étape de traduction de l'ARN de la première bibliothèque ; et
v) la sélection des fragments Fab d'intérêt provenant de la bibliothèque de fragments Fab.

2. Procédé selon la revendication 1, dans lequel la première bibliothèque comporte entre 10³ et 10⁵ éléments ou entre 10⁵ et 10¹⁶ éléments.

3. Procédé selon la revendication 1, dans lequel le système réactionnel d'affichage de ribosomes comprend un écran avec la puromycine ou un écran CIS.

4. Procédé selon la revendication 1, comprenant en outre le retrait du ribosome du système réactionnel d'affichage de ribosomes avant l'étape (v) consistant à sélectionner les fragments Fab d'intérêt.

5. Procédé selon la revendication 1, dans lequel l'étape de sélection comprend la capture des fragments Fab avec un antigène immobilisé.

6. Procédé selon la revendication 1, dans lequel les ARN des fragments Fab choisis dans l'étape (v) sont amplifiés et les produits amplifiés sont combinés à une bibliothèque et un système réactionnel d'affichage de ribosomes acellulaires pour générer une population de complexes comprenant un brin d'ARN, un ribosome et des fragments Fab qui sont enrichis en vue d'une liaison à l'antigène utilisé pour capturer les fragments Fab de l'étape (v).

7. Procédé selon la revendication 1, dans lequel le système de synthèse de protéines acellulaires comprend
(a) une réaction de phosphorylation oxydative produisant l'ATP ;
(b) un système de ribosomes reconstitués ; ou
(c) un extrait bactérien avec des co-facteurs associés.
